# EUROPEAN PATENT SPECIFICATION

(11) **EP 3 231 354 B1**
(45) Date of publication and mention of the grant of the patent: **17.03.2021**
(21) Application number: 16177762.8
(22) Date of filing: 04.07.2016
(51) Int. Cl.: A61B 5/00, A61B 5/11, A63B 23/02, A44B 11/02, A41F 9/02, A61F 5/02, A61B 5/08, A61B 5/107, A61B 5/113

(54) **SMART BELT**
INTELLIGENTES BAND
COURROIE À PUCE

(30) Priority: 11.04.2016 KR 20160044243
(43) Date of publication of application: 18.10.2017
(73) Proprietor: LG ELECTRONICS INC., Yeongdeungpo-gu Seoul 07336 (KR)
(72) Inventor: HONG, Jangwoo, 06772 Seoul (KR); JIN, Dongchul, 06772 Seoul (KR); LEE, Hyunok, 06772 Seoul (KR); PARK, Mihyun, 06772 Seoul (KR)
(74) Representative: Plasseraud IP

(56) References cited:
- WO-A2-2013/191933
- US-A- 6 146 312
- US-A1- 2007 066 462
- Chip Chick: "New Diet Belt watches your waistline - the Belty", You Tube, 11 January 2015 (2015-01-11), page 1, XP054977670, Retrieved from the Internet: URL:https://www.youtube.com/watch?v=2SMlbY hrgOo [retrieved on 2017-08-25]

## Description

### BACKGROUND OF THE INVENTION

### 1. Field of the Invention

This specification relates to a smart belt, and more particularly, to a smart belt capable of providing a feedback to a user in a manner of tightening/loosening the smart belt in response to the user's motion (activity) or state.

### 2. Background of the Invention

Terminals may be divided into mobile/portable terminals and stationary terminals according to their mobility. Also, the mobile terminals may be classified into handheld terminals and vehicle mount terminals according to whether or not a user can directly carry them.

Mobile terminals have become increasingly more functional. Examples of such functions include data and voice communications, capturing images and video via a camera, recording audio, playing music files via a speaker system, and displaying images and video on a display. Some mobile terminals include additional functionality which supports game playing, while other terminals are configured as multimedia players. More recently, mobile terminals have been configured to receive broadcast and multicast signals which permit viewing of content such as videos and television programs.

As it becomes multifunctional, a mobile terminal can be allowed to capture still images or moving images, play music or video files, play games, receive broadcast and the like, so as to be implemented as an integrated multimedia player.

In recent time, various wearable devices, such as smart glasses, smart watches and smart belts, operable with a mobile terminal are developed and put on the market. Among others, the smart belt is a wearable device with a sensor attached to a belt (strap) and can sense a condition or shape of a user's body through the mounted sensor.

However, smart belts which are currently on sale merely perform simple functions, such as adjusting a belt length according to a user's physical shape or checking a state of the user's health based on the change of the belt length. However, various functions and applying methods using the smart belts have not been provided.

In addition, detailed studies on operations of the smart belt according to a condition or shape of the user's body are required.

US2007/066462 discloses an exercise belt for stomach muscles having a belt with a toothed rack that engages with a pinion wheel to provide tension to the belt. WO2013/191933 discloses a device for providing support to a part of the human body with a tensionable strap. Tension can be applied to the strap via a system of pulley wheels and a cord.

### SUMMARY OF THE INVENTION

Therefore, an aspect of the detailed description is to provide a smart belt capable of tightening or loosening a strap by recognizing an applied force, capable of providing a feedback to a user in a manner of tightening/loosening the smart belt according to an activity mode.

To achieve these and other advantages and in accordance with the purpose of this specification, as embodied and broadly described herein, there is provided a smart belt according to claim 1
In accordance with an embodiment disclosed herein, the second shaft may be connected to a driving motor driving the second shaft. The second gear may be a worm gear, and the first gear may be a spur gear intersecting with the second gear.

In accordance with an embodiment disclosed herein, the second gear may be provided with a thread formed in a spiral direction, and an angle that the thread is formed may be less than 45° from a direction perpendicular to the second shaft.

In accordance with the invention disclosed herein, the third shaft has both ends fixed to inner side walls of the body, and the strap is closely adhered to overlap the third gear and the fourth gear.

In accordance with the invention disclosed herein, the third gear is arranged between the pair of fourth gears that are spaced apart from each other.

In accordance with an embodiment disclosed herein, the smart belt may further include a pair of supporting members provided on both end portions of the first shaft and the third shaft, one of the pair of supporting members having a through hole formed therethrough such that the first shaft is inserted through the through hole to be externally exposed, and fixing one end portion of the third shaft, a slide button provided on at least one end portion of the first shaft and configured to selectively separate the first gear and the second gear from each other, and a first elastic member provided on an end portion of the first shaft and configured to apply a restoring force to the first shaft.

In accordance with the invention disclosed herein, the third gear and the fourth gear include metal members provided on outer circumferences of the first shaft and the third shaft, respectively, and rubber members provided on outer circumferences of the metal members, respectively.

In accordance with an embodiment disclosed herein, the cover may be provided with a protrusion protruding from one surface thereof toward the body, and the protrusion may be brought into contact with an inner side surface of the body.

In accordance with an embodiment disclosed herein, the smart belt may further include a main circuit board provided in the body, a battery disposed at one side of the main circuit board, a vibration motor provided at one side of the battery and configured to generate vibration.

In accordance with an embodiment disclosed herein, the movement-detecting sensor may be one of an image sensor, a hall sensor or a resistance-measurement sensor. The strap may be provided with magnetic members when the movement-detecting sensor is the hall sensor, and provided with a conductive member when the movement-detecting sensor is the resistance-measurement sensor.

In accordance with an embodiment disclosed herein, the smart belt may further include a pair of doors coupled to both end portions of the through hole and rotatable toward an inner side of the body, and second elastic members provided at the pair of doors, respectively, to apply restoring forces to the pair of doors.

In accordance with an embodiment disclosed herein, the second elastic member may be a torsion spring.

In accordance with an embodiment disclosed herein, the strap may be made of a urethane material.

In accordance with one embodiment disclosed herein, a size of a buckle unit can be minimized by user of a worm gear, and simultaneously a change in a rotational shaft to which a force is transferred may be enabled.

In accordance with one embodiment disclosed herein, a reverse rotation can be prevented and thus the belt can be prevented from being unexpectedly loosened. In more detail, when desiring to manually move a strap, a contact between the strap and gears can be prevented, which may result in controlling a moving direction of the strap in a manual manner.

In accordance with one embodiment disclosed herein, the strap can be stably supported at three points, and an unevenness of a thickness of the strap caused due to a processing deviation of the strap can be flexibly solved.

In addition, in accordance with one embodiment disclosed herein, rubber members formed on outer circumferences of gears may be formed to be skewed, so as to increase a frictional force against the strap. Also, the strap may be made of a urethane material, and thus a frictional force between the strap and the rubber members can increase.

In accordance with one embodiment disclosed herein, an activity mode may be set by detecting user's posture and motion through a plurality of sensors provided in the smart belt, and the smart belt can tighten/loosen itself based on the set activity mode so as to be adjusted into a size suitable for the user. This may allow the user's posture to be corrected into a posture which is suitable for the user's current condition or desired by the user.

### BRIEF DESCRIPTION OF THE DRAWINGS

The accompanying drawings, which are included to provide a further understanding of the invention and are incorporated in and constitute a part of this specification, illustrate exemplary embodiments and together with the description serve to explain the principles of the invention.

In the drawings:
FIG. 1A is a block diagram of a mobile terminal in accordance with the present invention;
FIGS. 1B and 1C are conceptual views illustrating one example of a typical mobile terminal, viewed from different directions;
FIG. 2 is a schematic perspective view of a smart belt in accordance with one embodiment of the present invention;
FIG. 3 is a view illustrating an inner structure of a buckle unit in accordance with one embodiment of the present invention;
FIG. 4A is a perspective view of a body in accordance with one embodiment of the present invention;
FIG. 4B is a perspective view of a gear unit in accordance with one embodiment of the present invention;
FIG. 4C is a perspective view of a cover in accordance with one embodiment of the present invention;
FIG. 5 is a view illustrating a state that a strap is inserted in a buckle unit in accordance with one embodiment of the present invention;
FIG. 6 is an enlarged perspective view of a region B of FIG. 5;
FIG. 7A is a view illustrating a shape that a first shaft and a second shaft intersect with each other in accordance with one embodiment of the present invention;
FIG. 7B is a schematic view of forces applied to the first shaft and the second shaft;
FIG. 8 is a view illustrating a configuration of allowing a manual manipulation of a smart belt in accordance with one embodiment of the present invention;
FIGS. 9A and 9B are views illustrating a state that a strap is fixed or released by a gear unit in accordance with one embodiment of the present invention;
FIG. 10 is a view illustrating an arrangement of gears which overlap a strap in accordance with one embodiment of the present invention;
FIG. 11 is a schematic view illustrating a position of a force applied to a strap in accordance with one embodiment of the present invention;
FIG. 12 is a perspective view of a gear in accordance with one embodiment of the present invention;
FIGS. 13A to 13C are cross-sectional views illustrating an inserted state of a strap in accordance with an exemplary variation;
FIG. 14A is a view illustrating a state before a strap is inserted into a body in accordance with one embodiment of the present invention;
FIG. 14B is a sectional view illustrating a state that the strap has been inserted in the body;
FIGS. 15A to 15C are views illustrating a sensor for detecting a movement of a strap in accordance with one embodiment of the present invention;
FIG. 16 is a flowchart illustrating a method of controlling a smart belt in accordance with one embodiment of the present invention;
FIG. 17 is a view illustrating an internal configuration of a smart belt in accordance with one embodiment of the present invention;
FIG. 18 is a flowchart illustrating an embodiment of using a smart belt in accordance with one embodiment of the present invention.

### DETAILED DESCRIPTION OF THE INVENTION

Description will now be given in detail according to exemplary embodiments disclosed herein, with reference to the accompanying drawings. For the sake of brief description with reference to the drawings, the same or equivalent components may be provided with the same or similar reference numbers, and description thereof will not be repeated. In general, a suffix such as "module" and "unit" may be used to refer to elements or components. Use of such a suffix herein is merely intended to facilitate description of the specification, and the suffix itself is not intended to give any special meaning or function. In the present invention, that which is well-known to one of ordinary skill in the relevant art has generally been omitted for the sake of brevity. The accompanying drawings are used to help easily understand various technical features and it should be understood that the embodiments presented herein are not limited by the accompanying drawings. As such, the present invention should be construed to extend to any alterations, equivalents and substitutes in addition to those which are particularly set out in the accompanying drawings.

It will be understood that although the terms first, second, etc. may be used herein to describe various elements, these elements should not be limited by these terms. These terms are generally only used to distinguish one element from another.

It will be understood that when an element is referred to as being "connected with" another element, the element can be connected with the other element or intervening elements may also be present. In contrast, when an element is referred to as being "directly connected with" another element, there are no intervening elements present.

A singular representation may include a plural representation unless it represents a definitely different meaning from the context.

Terms such as "include" or "has" are used herein and should be understood that they are intended to indicate an existence of several components, functions or steps, disclosed in the specification, and it is also understood that greater or fewer components, functions, or steps may likewise be utilized.

Mobile terminals presented herein may be implemented using a variety of different types of terminals. Examples of such terminals include cellular phones, smart phones, user equipment, laptop computers, digital broadcast terminals, personal digital assistants (PDAs), portable multimedia players (PMPs), navigators, portable computers (PCs), slate PCs, tablet PCs, ultra books, wearable devices (for example, smart watches, smart glasses, head mounted displays (HMDs)), and the like.

By way of non-limiting example only, further description will be made with reference to particular types of mobile terminals. However, such teachings apply equally to other types of terminals, such as those types noted above. In addition, these teachings may also be applied to stationary terminals such as digital TV, desktop computers, digital signage and the like.

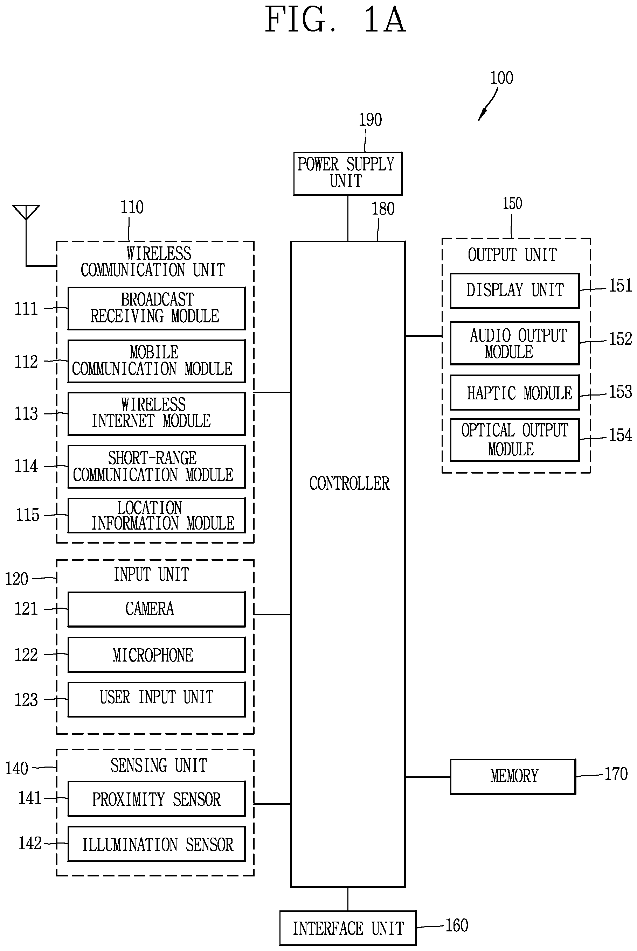

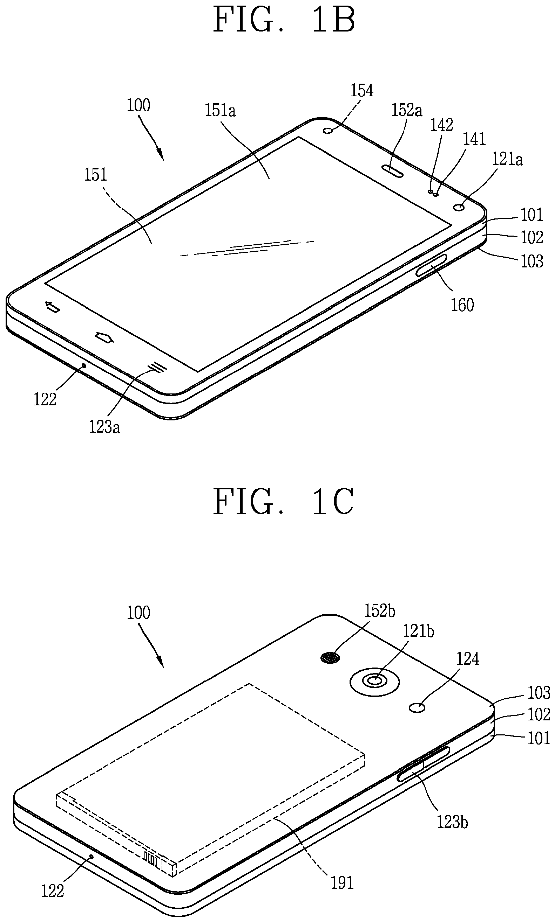

Reference is now made to FIGS. 1A-1C, where FIG. 1A is a block diagram of a mobile terminal in accordance with the present disclosure, and FIGS. 1B and 1C are conceptual views of one example of the mobile terminal, viewed from different directions.

The mobile terminal 100 is shown having components such as a wireless communication unit 110, an input unit 120, a sensing unit 140, an output unit 150, an interface unit 160, a memory 170, a controller 180, and a power supply unit 190. It is understood that implementing all of the illustrated components is not a requirement, and that greater or fewer components may alternatively be implemented.

In more detail, the wireless communication unit 110 may typically include one or more modules which permit communications such as wireless communications between the mobile terminal 100 and a wireless communication system, communications between the mobile terminal 100 and another mobile terminal, communications between the mobile terminal 100 and an external server. Further, the wireless communication unit 110 may typically include one or more modules which connect the mobile terminal 100 to one or more networks.

The wireless communication unit 110 may include one or more of a broadcast receiving module 111, a mobile communication module 112, a wireless Internet module 113, a short-range communication module 114, and a location information module 115.

The input unit 120 may include a camera 121 or an image input unit for obtaining images or video, a microphone 122, which is one type of audio input device for inputting an audio signal, and a user input unit 123 (for example, a touch key, a mechanical key, and the like) for allowing a user to input information. Data (for example, audio, video, image, and the like) may be obtained by the input unit 120 and may be analyzed and processed according to user commands.

The sensing unit 140 may typically be implemented using one or more sensors configured to sense internal information of the mobile terminal, the surrounding environment of the mobile terminal, user information, and the like. For example, the sensing unit 140 may include at least one of a proximity sensor 141, an illumination sensor 142, a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 121), a microphone 122, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like). The mobile terminal disclosed herein may be configured to utilize information obtained from one or more sensors of the sensing unit 140, and combinations thereof.

The output unit 150 may typically be configured to output various types of information, such as audio, video, tactile output, and the like. The output unit 150 may be shown having at least one of a display unit 151, an audio output module 152, a haptic module 153, and an optical output module 154. The display unit 151 may have an inter-layered structure or an integrated structure with a touch sensor in order to facilitate a touch screen. The touch screen may provide an output interface between the mobile terminal 100 and a user, as well as function as the user input unit 123 which provides an input interface between the mobile terminal 100 and the user.

The interface unit 160 serves as an interface with various types of external devices that can be coupled to the mobile terminal 100. The interface unit 160, for example, may include any of wired or wireless ports, external power supply ports, wired or wireless data ports, memory card ports, ports for connecting a device having an identification module, audio input/output (I/O) ports, video I/O ports, earphone ports, and the like. In some cases, the mobile terminal 100 may perform assorted control functions associated with a connected external device, in response to the external device being connected to the interface unit 160.

The memory 170 is typically implemented to store data to support various functions or features of the mobile terminal 100. For instance, the memory 170 may be configured to store application programs executed in the mobile terminal 100, data or instructions for operations of the mobile terminal 100, and the like. Some of these application programs may be downloaded from an external server via wireless communication. Other application programs may be installed within the mobile terminal 100 at time of manufacturing or shipping, which is typically the case for basic functions of the mobile terminal 100 (for example, receiving a call, placing a call, receiving a message, sending a message, and the like). It is common for application programs to be stored in the memory 170, installed in the mobile terminal 100, and executed by the controller 180 to perform an operation (or function) for the mobile terminal 100.

The controller 180 typically functions to control overall operation of the mobile terminal 100, in addition to the operations associated with the application programs. The controller 180 may provide or process information or functions appropriate for a user by processing signals, data, information and the like, which are input or output by the aforementioned various components, or activating application programs stored in the memory 170.

Also, the controller 180 controls some or all of the components illustrated in FIG. 1A according to the execution of an application program that have been stored in the memory 170. In addition, the controller 180 may control at least two of those components included in the mobile terminal to activate the application program.

The power supply unit 190 can be configured to receive external power or provide internal power in order to supply appropriate power required for operating elements and components included in the mobile terminal 100. The power supply unit 190 may include a battery, and the battery may be configured to be embedded in the terminal body, or configured to be detachable from the terminal body.

At least part of the components may cooperatively operate to implement an operation, a control or a control method of a mobile terminal according to various embodiments disclosed herein. Also, the operation, the control or the control method of the mobile terminal may be implemented on the mobile terminal by an activation of at least one application program stored in the memory 170.

Hereinafter, description will be given in more detail of the aforementioned components with reference to FIG. 1A, prior to describing various embodiments implemented through the mobile terminal 100.

First, regarding the wireless communication unit 110, the broadcast receiving module 111 is typically configured to receive a broadcast signal and/or broadcast associated information from an external broadcast managing entity via a broadcast channel. The broadcast channel may include a satellite channel, a terrestrial channel, or both. In some embodiments, two or more broadcast receiving modules 111 may be utilized to facilitate simultaneously receiving of two or more broadcast channels, or to support switching among broadcast channels.

The mobile communication module 112 can transmit and/or receive wireless signals to and from one or more network entities. Typical examples of a network entity include a base station, an external mobile terminal, a server, and the like. Such network entities form part of a mobile communication network, which is constructed according to technical standards or communication methods for mobile communications (for example, Global System for Mobile Communication (GSM), Code Division Multi Access (CDMA), CDMA2000(Code Division Multi Access 2000), Enhanced Voice-Date Optimized or Enhanced Voice-Data Only (EV-DO), Wideband CDMA (WCDMA), High Speed Downlink Packet access (HSDPA), High Speed Uplink Packet Access (HSUPA), Long Term Evolution (LTE), LTE-advanced (LTE-A) and the like).

Examples of the wireless signals include audio call signals, video (telephony) call signals, or various formats of data to support communication of text and multimedia messages.

The wireless Internet module 113 is configured to facilitate wireless Internet access. This module may be internally or externally coupled to the mobile terminal 100. The wireless Internet module 113 may transmit and/or receive wireless signals via communication networks according to wireless Internet technologies.

Examples of such wireless Internet access include Wireless LAN (WLAN), Wireless Fidelity (Wi-Fi), Wi-Fi Direct, Digital Living Network Alliance (DLNA), Wireless Broadband (WiBro), Worldwide Interoperability for Microwave Access (WiMAX), High Speed Downlink Packet Access (HSDPA), High Speed Uplink Packet Access (HSUPA), Long Term Evolution (LTE), LTE-advanced (LTE-A) and the like. The wireless Internet module 113 may transmit/receive data according to one or more of such wireless Internet technologies, and other Internet technologies as well.

In some embodiments, when the wireless Internet access is implemented according to, for example, WiBro, HSDPA, HSUPA, GSM, CDMA, WCDMA, LTE, LET-A, and the like, as part of a mobile communication network, the wireless Internet module 113 may be understood as a type of the mobile communication module 112.

The short-range communication module 114 is configured to facilitate short-range communications. Suitable technologies for implementing such short-range communications include BLUETOOTHTM, Radio Frequency IDentification (RFID), Infrared Data Association (IrDA), Ultra-WideBand (UWB), ZigBee, Near Field Communication (NFC), Wireless-Fidelity (Wi-Fi), Wi-Fi Direct, Wireless USB (Wireless Universal Serial Bus), and the like. The short-range communication module 114 in general supports wireless communications between the mobile terminal 100 and a wireless communication system, communications between the mobile terminal 100 and another mobile terminal 100, or communications between the mobile terminal and a network where another mobile terminal 100 (or an external server) is located, via wireless area networks. One example of the wireless area networks is a wireless personal area networks.

Here, another mobile terminal (which may be configured similarly to mobile terminal 100) may be a wearable device, for example, a smart watch, a smart glass or a head mounted display (HMD), which is able to exchange data with the mobile terminal 100 (or otherwise cooperate with the mobile terminal 100). The short-range communication module 114 may sense or recognize the wearable device, and permit communication between the wearable device and the mobile terminal 100. In addition, when the sensed wearable device is a device which is authenticated to communicate with the mobile terminal 100, the controller 180, for example, may cause transmission of at least part of data processed in the mobile terminal 100 to the wearable device via the short-range communication module 114. Hence, a user of the wearable device may use the data processed in the mobile terminal 100 on the wearable device. For example, when a call is received in the mobile terminal 100, the user may answer the call using the wearable device. Also, when a message is received in the mobile terminal 100, the user can check the received message using the wearable device.

The location information module 115 is generally configured to detect, calculate, derive or otherwise identify a position (or current position) of the mobile terminal. As an example, the location information module 115 includes a Global Position System (GPS) module, a Wi-Fi module, or both. For example, when the mobile terminal uses a GPS module, a position of the mobile terminal may be acquired using a signal sent from a GPS satellite. As another example, when the mobile terminal uses the Wi-Fi module, a position of the mobile terminal can be acquired based on information related to a wireless access point (AP) which transmits or receives a wireless signal to or from the Wi-Fi module. If desired, the location information module 115 may alternatively or additionally function with any of the other modules of the wireless communication unit 110 to obtain data related to the position of the mobile terminal. The location information module 115 is a module used for acquiring the position (or the current position) and may not be limited to a module for directly calculating or acquiring the position of the mobile terminal.

The input unit 120 may be configured to permit various types of inputs to the mobile terminal 100. Examples of such inputs include audio, image, video, data, and user input. Image and video input is often obtained using one or more cameras 121. Such cameras 121 may process image frames of still pictures or video obtained by image sensors in a video or image capture mode. The processed image frames can be displayed on the display unit 151 or stored in memory 170. Meanwhile, the cameras 121 may be arranged in a matrix configuration to permit a plurality of images having various angles or focal points to be input to the mobile terminal 100. Also, the cameras 121 may be located in a stereoscopic arrangement to acquire left and right images for implementing a stereoscopic image.

The microphone 122 processes an external audio signal into electric audio (sound) data. The processed audio data can be processed in various manners according to a function being executed in the mobile terminal 100. If desired, the microphone 122 may include assorted noise removing algorithms to remove unwanted noise generated in the course of receiving the external audio signal.

The user input unit 123 is a component that permits input by a user. Such user input may enable the controller 180 to control operation of the mobile terminal 100. The user input unit 123 may include one or more of a mechanical input element (for example, a mechanical key, a button located on a front and/or rear surface or a side surface of the mobile terminal 100, a dome switch, a jog wheel, a jog switch, and the like), or a touch-sensitive input element, among others. As one example, the touch-sensitive input element may be a virtual key, a soft key or a visual key, which is displayed on a touch screen through software processing, or a touch key which is located on the mobile terminal at a location that is other than the touch screen. On the other hand, the virtual key or the visual key may be displayed on the touch screen in various shapes, for example, graphic, text, icon, video, or a combination thereof.

The sensing unit 140 is generally configured to sense one or more of internal information of the mobile terminal, surrounding environment information of the mobile terminal, user information, or the like, and generate a corresponding sensing signal. The controller 180 generally cooperates with the sending unit 140 to control operation of the mobile terminal 100 or execute data processing, a function or an operation associated with an application program installed in the mobile terminal based on the sensing signal. The sensing unit 140 may be implemented using any of a variety of sensors, some of which will now be described in more detail.

The proximity sensor 141 refers to a sensor to sense presence or absence of an object approaching a surface, or an object located near a surface, by using an electromagnetic field, infrared rays, or the like without a mechanical contact. The proximity sensor 141 may be arranged at an inner region of the mobile terminal covered by the touch screen, or near the touch screen.

The proximity sensor 141, for example, may include any of a transmissive type photoelectric sensor, a direct reflective type photoelectric sensor, a mirror reflective type photoelectric sensor, a high-frequency oscillation proximity sensor, a capacitance type proximity sensor, a magnetic type proximity sensor, an infrared rays proximity sensor, and the like. When the touch screen is implemented as a capacitance type, the proximity sensor 141 can sense proximity of a pointer relative to the touch screen by changes of an electromagnetic field, which is responsive to an approach of an object with conductivity. In this case, the touch screen (touch sensor) may also be categorized as a proximity sensor.

The term "proximity touch" will often be referred to herein to denote the scenario in which a pointer is positioned to be proximate to the touch screen without contacting the touch screen. The term "contact touch" will often be referred to herein to denote the scenario in which a pointer makes physical contact with the touch screen. For the position corresponding to the proximity touch of the pointer relative to the touch screen, such position will correspond to a position where the pointer is perpendicular to the touch screen. The proximity sensor 141 may sense proximity touch, and proximity touch patterns (for example, distance, direction, speed, time, position, moving status, and the like). In general, controller 180 processes data corresponding to proximity touches and proximity touch patterns sensed by the proximity sensor 141, and cause output of visual information on the touch screen. In addition, the controller 180 can control the mobile terminal 100 to execute different operations or process different data (or information) according to whether a touch with respect to a point on the touch screen is either a proximity touch or a contact touch.

A touch sensor can sense a touch (or a touch input) applied to the touch screen, such as display unit 151, using any of a variety of touch methods. Examples of such touch methods include a resistive type, a capacitive type, an infrared type, and a magnetic field type, among others.

As one example, the touch sensor may be configured to convert changes of pressure applied to a specific part of the display unit 151, or convert capacitance occurring at a specific part of the display unit 151, into electric input signals. The touch sensor may also be configured to sense not only a touched position and a touched region, but also touch pressure and/or touch capacitance. A touch object is generally used to apply a touch input to the touch sensor. Examples of typical touch objects include a finger, a touch pen, a stylus pen, a pointer, or the like.

When a touch input is sensed by a touch sensor, corresponding signals may be transmitted to a touch controller. The touch controller may process the received signals, and then transmit corresponding data to the controller 180. Accordingly, the controller 180 may sense which region of the display unit 151 has been touched. Here, the touch controller may be a component separate from the controller 180, the controller 180, and combinations thereof.

Meanwhile, the controller 180 may execute the same or different controls according to a type of touch object that touches the touch screen or a touch key provided in addition to the touch screen. Whether to execute the same or different control according to the object which provides a touch input may be decided based on a current operating state of the mobile terminal 100 or a currently executed application program, for example.

The touch sensor and the proximity sensor may be implemented individually, or in combination, to sense various types of touches. Such touches includes a short (or tap) touch, a long touch, a multi-touch, a drag touch, a flick touch, a pinch-in touch, a pinch-out touch, a swipe touch, a hovering touch, and the like.

If desired, an ultrasonic sensor may be implemented to recognize position information relating to a touch object using ultrasonic waves. The controller 180, for example, may calculate a position of a wave generation source based on information sensed by an illumination sensor and a plurality of ultrasonic sensors. Since light is much faster than ultrasonic waves, the time for which the light reaches the optical sensor is much shorter than the time for which the ultrasonic wave reaches the ultrasonic sensor. The position of the wave generation source may be calculated using this fact. For instance, the position of the wave generation source may be calculated using the time difference from the time that the ultrasonic wave reaches the sensor based on the light as a reference signal.

The camera 121 typically includes at least one a camera sensor (CCD, CMOS etc.), a photo sensor (or image sensors), and a laser sensor.

Implementing the camera 121 with a laser sensor may allow detection of a touch of a physical object with respect to a 3D stereoscopic image. The photo sensor may be laminated on, or overlapped with, the mobile terminal. The photo sensor may be configured to scan movement of the physical object in proximity to the touch screen. In more detail, the photo sensor may include photo diodes and transistors (TR) at rows and columns to scan content received at the photo sensor using an electrical signal which changes according to the quantity of applied light. Namely, the photo sensor may calculate the coordinates of the physical object according to variation of light to thus obtain position information of the physical object.

The display unit 151 is generally configured to output information processed in the mobile terminal 100. For example, the display unit 151 may display execution screen information of an application program executing at the mobile terminal 100 or user interface (Ul) and graphic user interface (GUI) information in response to the execution screen information.

In some embodiments, the flexible display unit 151 may be implemented as a stereoscopic display unit for displaying stereoscopic images.

A typical stereoscopic display unit may employ a stereoscopic display scheme such as a stereoscopic scheme (a glass scheme), an auto-stereoscopic scheme (glassless scheme), a projection scheme (holographic scheme), or the like.

The audio output module 152 is generally configured to output audio data. Such audio data may be obtained from any of a number of different sources, such that the audio data may be received from the wireless communication unit 110 or may have been stored in the memory 170. The audio data may be output during modes such as a signal reception mode, a call mode, a record mode, a voice recognition mode, a broadcast reception mode, and the like. The audio output module 152 can provide audible output related to a particular function (e.g., a call signal reception sound, a message reception sound, etc.) performed by the mobile terminal 100. The audio output module 152 may also be implemented as a receiver, a speaker, a buzzer, or the like.

A haptic module 153 can be configured to generate various tactile effects that a user feels, perceive, or otherwise experience. A typical example of a tactile effect generated by the haptic module 153 is vibration. The strength, pattern and the like of the vibration generated by the haptic module 153 can be controlled by user selection or setting by the controller. For example, the haptic module 153 may output different vibrations in a combining manner or a sequential manner.

Besides vibration, the haptic module 153 can generate various other tactile effects, including an effect by stimulation such as a pin arrangement vertically moving to contact skin, a spray force or suction force of air through a jet orifice or a suction opening, a touch to the skin, a contact of an electrode, electrostatic force, an effect by reproducing the sense of cold and warmth using an element that can absorb or generate heat, and the like.

The haptic module 153 can also be implemented to allow the user to feel a tactile effect through a muscle sensation such as the user's fingers or arm, as well as transferring the tactile effect through direct contact. Two or more haptic modules 153 may be provided according to the particular configuration of the mobile terminal 100.

An optical output module 154 can output a signal for indicating an event generation using light of a light source. Examples of events generated in the mobile terminal 100 may include message reception, call signal reception, a missed call, an alarm, a schedule alarm, an email reception, information reception through an application, and the like.

A signal output by the optical output module 154 may be implemented in such a manner that the mobile terminal emits monochromatic light or light with a plurality of colors. The signal output may be terminated as the mobile terminal senses that a user has checked the generated event, for example.

The interface unit 160 serves as an interface for external devices to be connected with the mobile terminal 100. For example, the interface unit 160 can receive data transmitted from an external device, receive power to transfer to elements and components within the mobile terminal 100, or transmit internal data of the mobile terminal 100 to such external device. The interface unit 160 may include wired or wireless headset ports, external power supply ports, wired or wireless data ports, memory card ports, ports for connecting a device having an identification module, audio input/output (I/O) ports, video I/O ports, earphone ports, or the like.

The identification module may be a chip that stores various information for authenticating authority of using the mobile terminal 100 and may include a user identity module (UIM), a subscriber identity module (SIM), a universal subscriber identity module (USIM), and the like. In addition, the device having the identification module (also referred to herein as an "identifying device") may take the form of a smart card. Accordingly, the identifying device can be connected with the terminal 100 via the interface unit 160.

When the mobile terminal 100 is connected with an external cradle, the interface unit 160 can serve as a passage to allow power from the cradle to be supplied to the mobile terminal 100 or may serve as a passage to allow various command signals input by the user from the cradle to be transferred to the mobile terminal there through. Various command signals or power input from the cradle may operate as signals for recognizing that the mobile terminal is properly mounted on the cradle.

The memory 170 can store programs to support operations of the controller 180 and store input/output data (for example, phonebook, messages, still images, videos, etc.). The memory 170 may store data related to various patterns of vibrations and audio which are output in response to touch inputs on the touch screen.

The memory 170 may include one or more types of storage mediums including a flash memory type, a hard disk type, a solid state disk (SSD) type, a silicon disk drive (SDD) type, a multimedia card micro type, a card-type memory (e.g., SD or DX memory, etc), a Random Access Memory (RAM), a Static Random Access Memory (SRAM), a Read-Only Memory (ROM), an Electrically Erasable Programmable Read-Only Memory (EEPROM), a Programmable Read-Only memory (PROM), a magnetic memory, a magnetic disk, an optical disk, and the like. The mobile terminal 100 may also be operated in relation to a network storage device that performs the storage function of the memory 170 over a network, such as the Internet.

The controller 180 may typically control an operation associated with an application program and the general operations of the mobile terminal 100. For example, the controller 180 may set or release a lock state for restricting a user from inputting a control command with respect to applications when a status of the mobile terminal meets a preset condition.

The controller 180 can also perform the controlling and processing associated with voice calls, data communications, video calls, and the like, or perform pattern recognition processing to recognize a handwriting input or a picture drawing input performed on the touch screen as characters or images, respectively. In addition, the controller 180 can control one or a combination of those components in order to implement various exemplary embodiments disclosed herein.

The power supply unit 190 receives external power or provide internal power and supply the appropriate power required for operating respective elements and components included in the mobile terminal 100. The power supply unit 190 may include a battery, which is typically rechargeable or be detachably coupled to the terminal body for charging.

The power supply unit 190 may include a connection port. The connection port may be configured as one example of the interface unit 160 to which an external charger for supplying power to recharge the battery is electrically connected.

As another example, the power supply unit 190 may be configured to recharge the battery in a wireless manner without use of the connection port. In this example, the power supply unit 190 can receive power, transferred from an external wireless power transmitter, using at least one of an inductive coupling method which is based on magnetic induction or a magnetic resonance coupling method which is based on electromagnetic resonance.

Various embodiments described herein may be implemented in a computer-readable medium, a machine-readable medium, or similar medium using, for example, software, hardware, or any combination thereof.

Referring now to FIGS. 1B and 1C, the mobile terminal 100 is described with reference to a bar-type the body of the portable electronic device. However, the mobile terminal 100 may alternatively be implemented in any of a variety of different configurations. Examples of such configurations include watch-type, clip-type, glasses-type, or as a folder-type, flip-type, slide-type, swing-type, and swivel-type in which two and more bodies are combined with each other in a relatively movable manner, and combinations thereof. However, such teachings with regard to a particular type of portable electronic device will generally apply to other types of mobile terminals as well.

The body of the mobile terminal may be understood to indicate the mobile terminal 100 by regarding the mobile terminal 100 as at least one assembly.

The mobile terminal 100 will generally include a case (for example, frame, housing, cover, and the like) forming the appearance of the terminal. In this embodiment, the case is formed using a front case 101 and a rear case 102. Various electronic components are incorporated into a space formed between the front case 101 and the rear case 102. At least one middle case may be additionally positioned between the front case 101 and the rear case 102.

The display unit 151 is shown located on the front side of the terminal body to output information. As illustrated, a window 151a of the display unit 151 may be mounted to the front case 101 to form the front surface of the terminal body together with the front case 101.

In some embodiments, electronic components may also be mounted to the rear case 102. Examples of such electronic components include a detachable battery 191, an identification module, a memory card, and the like. Rear cover 103 is shown covering the electronic components, and this cover may be detachably coupled to the rear case 102. Therefore, when the rear cover 103 is detached from the rear case 102, the electronic components mounted to the rear case 102 are externally exposed.

As illustrated, when the rear cover 103 is coupled to the rear case 102, a side surface of the rear case 102 is partially exposed. In some cases, upon the coupling, the rear case 102 may also be completely shielded by the rear cover 103. In some embodiments, the rear cover 103 may include an opening for externally exposing a camera 121b or an audio output module 152b.

The cases 101, 102, 103 may be formed by injection-molding synthetic resin or may be formed of a metal, for example, stainless steel (STS), aluminum (Al), titanium (Ti), or the like.

As an alternative to the example in which the plurality of cases form an inner space for accommodating components, the mobile terminal 100 may be configured such that one case forms the inner space. In this example, a mobile terminal 100 having a unibody is formed in such a manner that synthetic resin or metal extends from a side surface to a rear surface.

If desired, the mobile terminal 100 may include a waterproofing unit (not shown) for preventing introduction of water into the terminal body. For example, the waterproofing unit may include a waterproofing member which is located between the window 151a and the front case 101, between the front case 101 and the rear case 102, or between the rear case 102 and the rear cover 103, to hermetically seal an inner space when those cases are coupled.

The mobile terminal 100 may include a display unit 151, first and second audio output module 152a and 152b, a proximity sensor 141, an illumination sensor 142, an optical output module 154, first and second cameras 121a and 121b, first and second manipulation units 123a and 123b, a microphone 122, an interface unit 160, and the like.

Hereinafter, as illustrated in FIGS. 1B and 1C, description will be given of the exemplary mobile terminal 100 in which the front surface of the terminal body is shown having the display unit 151, the first audio output module 152a, the proximity sensor 141, the illumination sensor 142, the optical output module 154, the first camera 121a, and the first manipulation unit 123a, the side surface of the terminal body is shown having the second manipulation unit 123b, the microphone 122, and the interface unit 160, and the rear surface of the terminal body is shown having the second audio output module 152b and the second camera 121b.

However, those components may not be limited to the arrangement. Some components may be omitted or rearranged or located on different surfaces. For example, the first manipulation unit 123a may be located on another surface of the terminal body, and the second audio output module 152b may be located on the side surface of the terminal body other than the rear surface of the terminal body.

The display unit 151 outputs information processed in the mobile terminal 100. For example, the display unit 151 may display execution screen information of an application program executing at the mobile terminal 100 or user interface (Ul) and graphic user interface (GUI) information in response to the execution screen information.

The display unit 151 may be implemented using one or more suitable display devices. Examples of such suitable display devices include a liquid crystal display (LCD), a thin film transistor-liquid crystal display (TFT-LCD), an organic light emitting diode (OLED), a flexible display, a 3-dimensional (3D) display, an e-ink display, and combinations thereof.

The display unit 151 may be implemented using two display devices, which can implement the same or different display technology. For instance, a plurality of the display units 151 may be arranged on one side, either spaced apart from each other, or these devices may be integrated, or these devices may be arranged on different surfaces.

The display unit 151 may also include a touch sensor which senses a touch input received at the display unit. When a touch is input to the display unit 151, the touch sensor may be configured to sense this touch and the controller 180, for example, may generate a control command or other signal corresponding to the touch. The content which is input in the touching manner may be a text or numerical value, or a menu item which can be indicated or designated in various modes.

The touch sensor may be configured in a form of a film having a touch pattern, disposed between the window 151a and a display on a rear surface of the window 151a, or a metal wire which is patterned directly on the rear surface of the window 151a. Alternatively, the touch sensor may be integrally formed with the display. For example, the touch sensor may be disposed on a substrate of the display or within the display.

The display unit 151 may also form a touch screen together with the touch sensor. Here, the touch screen may serve as the user input unit 123 (see FIG. 1A). Therefore, the touch screen may replace at least some of the functions of the first manipulation unit 123a.

The first audio output module 152a may be implemented in the form of a receiver for transferring call sounds to a user's ear and the second audio output module 152b may be implemented in the form of a loud speaker to output alarm sounds, multimedia audio reproduction, and the like.

The window 151a of the display unit 151 will typically include an aperture to permit audio generated by the first audio output module 152a to pass. One alternative is to allow audio to be released along an assembly gap between the structural bodies (for example, a gap between the window 151a and the front case 101). In this case, a hole independently formed to output audio sounds may not be seen or is otherwise hidden in terms of appearance, thereby further simplifying the appearance and manufacturing of the mobile terminal 100.

The optical output module 154 can be configured to output light for indicating an event generation. Examples of such events include a message reception, a call signal reception, a missed call, an alarm, a schedule alarm, an email reception, information reception through an application, and the like. When a user has checked a generated event, the controller 180 can control the optical output module 154 to stop the light output.

The first camera 121a can process image frames such as still or moving images obtained by the image sensor in a capture mode or a video call mode. The processed image frames can then be displayed on the display unit 151 or stored in the memory 170.

The first and second manipulation units 123a and 123b are examples of the user input unit 123, which may be manipulated by a user to provide input to the mobile terminal 100. The first and second manipulation units 123a and 123b may also be commonly referred to as a manipulating portion, and may employ any tactile method that allows the user to perform manipulation such as touch, push, scroll, or the like. The first and second manipulation units 123a and 123b may also employ any non-tactile method that allows the user to perform manipulation such as proximity touch, hovering, or the like.

FIG. 1B illustrates the first manipulation unit 123a as a touch key, but possible alternatives include a mechanical key, a push key, a touch key, and combinations thereof.

Input received at the first and second manipulation units 123a and 123b may be used in various ways. For example, the first manipulation unit 123a may be used by the user to provide an input to a menu, home key, cancel, search, or the like, and the second manipulation unit 123b may be used by the user to provide an input to control a volume level being output from the first or second audio output modules 152a or 152b, to switch to a touch recognition mode of the display unit 151, or the like.

As another example of the user input unit 123, a rear input unit (not shown) may be located on the rear surface of the terminal body. The rear input unit can be manipulated by a user to provide input to the mobile terminal 100. The input may be used in a variety of different ways. For example, the rear input unit may be used by the user to provide an input for power on/off, start, end, scroll, control volume level being output from the first or second audio output modules 152a or 152b, switch to a touch recognition mode of the display unit 151, and the like. The rear input unit may be configured to permit touch input, a push input, or combinations thereof.

The rear input unit may be located to overlap the display unit 151 of the front side in a thickness direction of the terminal body. As one example, the rear input unit may be located on an upper end portion of the rear side of the terminal body such that a user can easily manipulate it using a forefinger when the user grabs the terminal body with one hand. Alternatively, the rear input unit can be positioned at most any location of the rear side of the terminal body.

When the rear input unit is provided at the rear surface of the terminal body, a new type of user interface using this may be implemented. Embodiments that include the rear input unit may implement some or all of the functionality of the first manipulation unit 123a in the rear input unit. As such, in situations where the first manipulation unit 123a is omitted from the front side, the display unit 151 can have a larger screen.

As a further alternative, the mobile terminal 100 may include a finger scan sensor which scans a user's fingerprint. The controller 180 can then use fingerprint information sensed by the finger scan sensor as part of an authentication procedure. The finger scan sensor may also be installed in the display unit 151 or implemented in the user input unit 123.

The microphone 122 is shown located at an end of the mobile terminal 100, but other locations are possible. If desired, multiple microphones may be implemented, with such an arrangement permitting the receiving of stereo sounds.

The interface unit 160 may serve as a path allowing the mobile terminal 100 to interface with external devices. For example, the interface unit 160 may include one or more of a connection terminal for connecting to another device (for example, an earphone, an external speaker, or the like), a port for near field communication (for example, an Infrared Data Association (IrDA) port, a BluetoothTM port, a wireless LAN port, and the like), or a power supply terminal for supplying power to the mobile terminal 100. The interface unit 160 may be implemented in the form of a socket for accommodating an external card, such as Subscriber Identification Module (SIM), User Identity Module (UIM), or a memory card for information storage.

The second camera 121b is shown located at the rear side of the terminal body and includes an image capturing direction that is substantially opposite to the image capturing direction of the first camera unit 121a.

The second camera 121b can include a plurality of lenses arranged along at least one line. The plurality of lenses may also be arranged in a matrix configuration. The cameras may be referred to as an "array camera." When the second camera 121b is implemented as an array camera, images may be captured in various manners using the plurality of lenses and images with better qualities.

A flash 124 is shown adjacent to the second camera 121b. When an image of a subject is captured with the camera 121b, the flash 124 may illuminate the subject.

The second audio output module 152b can be located on the terminal body. The second audio output module 152b may implement stereophonic sound functions in conjunction with the first audio output module 152a, and may be also used for implementing a speaker phone mode for call communication.

At least one antenna for wireless communication may be located on the terminal body. The antenna may be installed in the terminal body or formed by the case. For example, an antenna which configures a part of the broadcast receiving module 111 may be retractable into the terminal body. Alternatively, an antenna may be formed using a film attached to an inner surface of the rear cover 103, or a case that includes a conductive material.

A power supply unit 190 for supplying power to the mobile terminal 100 may include a battery 191, which is mounted in the terminal body or detachably coupled to an outside of the terminal body.

The battery 191 may receive power via a power source cable connected to the interface unit 160. Also, the battery 191 can be recharged in a wireless manner using a wireless charger. Wireless charging may be implemented by magnetic induction or electromagnetic resonance.

The rear cover 103 is shown coupled to the rear case 102 for shielding the battery 191, to prevent separation of the battery 191, and to protect the battery 191 from an external impact or from foreign material. When the battery 191 is detachable from the terminal body, the rear case 103 may be detachably coupled to the rear case 102.

An accessory for protecting an appearance or assisting or extending the functions of the mobile terminal 100 can also be provided on the mobile terminal 100. As one example of an accessory, a cover or pouch for covering or accommodating at least one surface of the mobile terminal 100 may be provided. The cover or pouch may cooperate with the display unit 151 to extend the function of the mobile terminal 100. Another example of the accessory is a touch pen for assisting or extending a touch input to a touch screen.

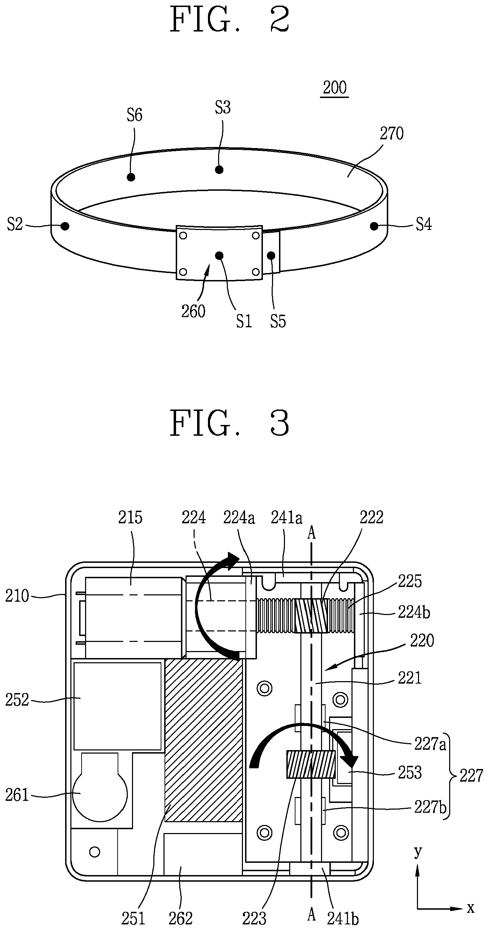

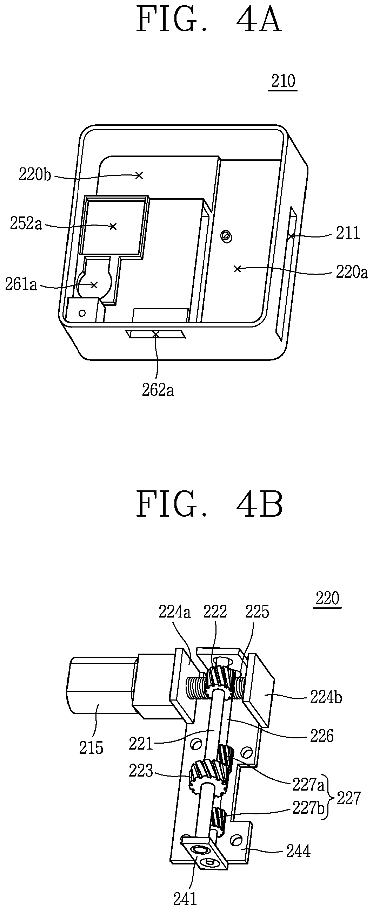

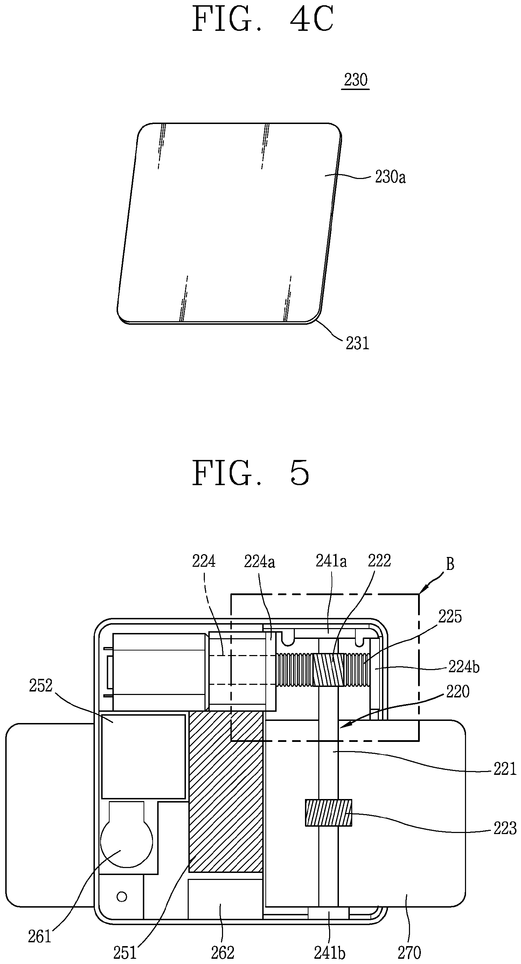

FIG. 2 is a schematic perspective view of a smart belt 200 in accordance with one exemplary embodiment, FIG. 3 is a view illustrating an inner structure of a buckle unit 260 in accordance with the one exemplary embodiment, viewed from a top, FIG. 4A is an inner perspective view of a body 210 in accordance with the one exemplary embodiment, FIG. 4B is a perspective view of a gear unit 220 in accordance with the one exemplary embodiment, and FIG. 4C is a perspective view of a cover 230 in accordance with the one exemplary embodiment.

Hereinafter, a smart belt in accordance with one exemplary embodiment of the present invention will be described with reference to FIGS. 2 to 4C.

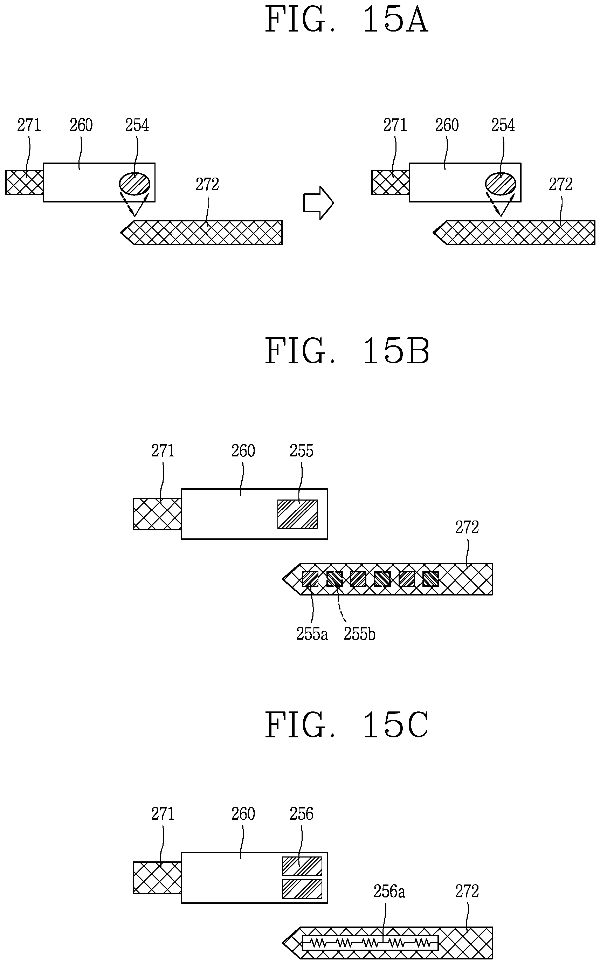

First, a state where a strap 270 is not inserted into the buckle unit 260 of the smart belt 200 is referred to as a first state, and a state where the strap 270 is inserted into the buckle unit 260 is referred to as a second state. One end portion of the strap 270 is coupled to the buckle unit 260 and another end portion of the strap 270 is selectively inserted into the buckle unit 260. Therefore, in the first state, the another end portion of the strap 270 forms a free end without being coupled to the buckle unit 260. On the other hand, in the second state, the another end portion of the strap 270 is coupled to the buckle unit 260 such that at least part of the strap 270 overlaps the buckle unit 260. In this instance, the one end portion of the strap 270 which is always fixed to the buckle unit 260 may be referred to as a fixed end portion 271 (see FIGS. 15A-15C), and the another end portion of the strap 270 is selectively inserted into the buckle unit 260 may be referred to as a free end portion 272 (see FIGS. 15A-15C).

For example, FIGS. 15A to 15C illustrate a process that the strap 270 is inserted into the buckle unit 260. A left drawing of FIG. 15A illustrates a state that the free end portion 272 starts to be inserted into the buckle unit 260. A right drawing of FIG. 15A illustrates a state that the free end portion 272 is inserted into the buckle unit 260 and thus at least part of the free end portion 272 is overlaid by the buckle unit 260 or the free end portion 271.

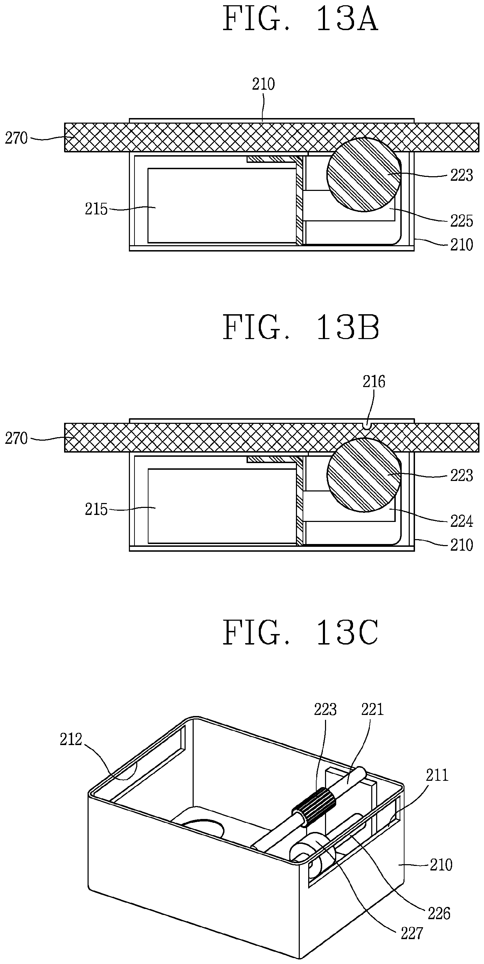

The smart belt 200 according to the one embodiment disclosed herein includes a body 210, a gear unit 220, and a cover 230. As illustrated in FIG. 3, the body 210, the gear unit 220 and the cover 230 form the buckle unit 260. The body 210 has a front surface externally exposed when the smart belt 200 is worn, and side surfaces extending from the front surface in a manner of intersecting with the front surface. The body 210, for example, may have an approximately rectangular parallelepiped shape with four side surfaces. In this instance, through holes 211 and 212 are formed through side surfaces facing each other, of the four side surfaces, such that the strap 270 moving in a first direction can be inserted therethrough. That is, the first and second through holes 211 and 212 are formed through side surfaces which intersect with the first direction, among the four side surfaces (see FIG. 13C).

In this instance, in the one embodiment of the present invention, the first direction refers to an x-axial direction and a second direction refers to a y-axial direction in FIG. 3.

The cover 230 which covers a rear surface of the body 210 is externally exposed, but is not exposed, when viewed from a front side, while the smart belt 200 is worn.

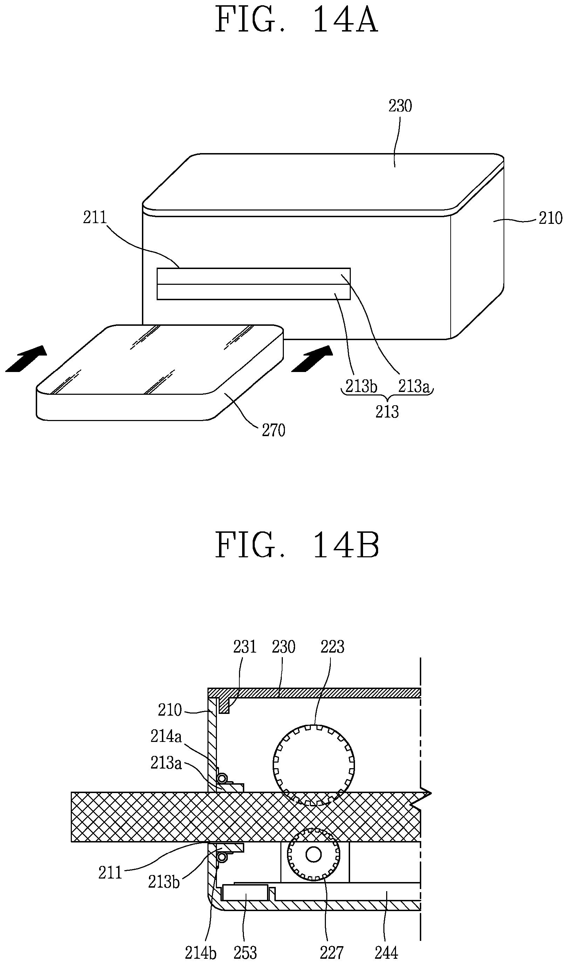

As illustrated in FIG. 4C, the cover 230 includes a front surface portion 230a exposed to outside. A protrusion 231 protruding toward the body 210 is formed on a rear surface of the front surface portion 230a. The protrusion 231 is brought into contact with an inner side surface of the body 210. In this manner, as the protrusion 231 is formed, external light which is likely to be introduced into the body 210 can be blocked (see FIG. 14B). This is to prevent other light, except for light reflected by the strap 270, from affecting a movement-detecting sensor 253, for example, an image sensor 254 because the image sensor 254 is disposed at a position adjacent to the first through hole 211 formed through the body 210.

The gear unit 220 is disposed in the body 210 and adjusts an amount that the strap 270 is tightened (i.e., an amount of tightening, tightening amount, etc.). The gear unit 220 includes a first shaft 221 formed in a second direction that intersects with the first direction and having a first gear 222, a second shaft 224 intersecting with the first shaft 221 and having a second gear 225 engaged (or gearing) with the first gear 222, and a third shaft 226 formed in parallel to the first shaft 221.

The second shaft 224 is connected to a driving motor 215 so as to obtain a rotational force generated by the driving motor 215, and the rotational force is transferred to the second gear 225 formed on the second shaft 224. The second gear 225 is formed on the second shaft 224. The second shaft 224 itself may be the second gear 225.

The first shaft 221 and the second shaft 224 intersect with each other. For example, the first shaft 221 and the second shaft 224 may be orthogonal to each other. That is, the second gear 225 may be a worm gear and the first gear 222 may be a worm wheel. The rotational force generated by the driving motor 215 is transferred from the second gear 225 to the first gear 222. In this instance, the second gear 225 may be a driving gear and the first gear 222 may be a driven gear which is driven by the second gear 225. Also, the first gear 222 may be a spur gear corresponding to the worm gear. However, the first and second gears 222 and 225 may not be specifically limited to the worm gear and the worm wheel, but may be implemented into any types if a force can be transferred in an intersecting direction.

In this manner, as the first shaft 221 and the second shaft 224 intersect with each other, the gear unit 220 may be reduced in volume and thus can be mounted in the body 210. That is, in the one exemplary embodiment disclosed herein, the worm gear is used to minimize a size of the buckle unit 260 and accordingly a rotation shaft to which a force is transferred is changeable.

In this instance, supporting plates 224a and 224b are provided at both ends of the second gear 225. The supporting plate 224b may be or may not be contactable with an inner wall of the body 210.

Also, the rotational force of the second shaft 224 is transferred to the first shaft 221 through the first and second gears 222 and 225, thereby adjusting an amount of tightening of the strap 270. This will be explained later.

The third shaft 226 is formed in parallel to the first shaft 221 with a predetermined gap from the first shaft 221. The strap 270 is disposed between the first shaft 221 and the third shaft 226 to be movable in the first direction. A third gear 223 which is spaced apart from the first gear 222 with a predetermined gap is formed on the first shaft 221. At least one fourth gear 227 is formed on the third shaft 226. The strap 270 is movable in a manner of coming in contact with the third gear 223 and the fourth gear 227. The fourth gear 227 is provided as a pair 227a and 227b. The pair of fourth gears 227a and 227b may have the same size and be made of the same material. The third gear 223 and the pair of fourth gears 227a and 227b may not necessarily be saw-toothed gears, and alternatively be rollers with a smooth outer circumference. That is, the third gear 223 and the fourth gears 227a and 227b may be implemented into any type or form if a frictional force Fs is generated against the strap 270 while the user wears the smart belt 200 and the frictional force Fs is greater than a force applied to the strap 270. However, it is preferable to use such saw-toothed gear in order to increase the frictional forces between the strap 270 and the third gear 223 and the fourth gears 227a and 227b.

The frictional force Fs between the strap 270 and the third gear 223 is equal to a frictional force Fg between the strap 270 and the fourth gears 227a and 227b. If those frictional forces Fs and Fg are not strong, the strap 270 may be easily drawn out of the buckle unit 260. That is, in the one exemplary embodiment disclosed herein, since a tensile force Ft is applied to the strap 270 by the user's body in the second state, if the tensile force Ft applied to the strap 270 is greater than the frictional forces Fs and Fg between the third gear 223 and the fourth gears 227a and 227b and the strap 270, the free end portion 272 of the strap 270 inserted into the buckle unit 260 may be easily drawn out of the buckle unit 260.

To prevent this, in the one embodiment disclosed herein, the third gear 223 and the fourth gears 227a 227b are implemented as the gears each having the saw-toothed shape.

Also, a force of pulling the strap 270 out is weak due to sufficiently strong frictional forces Fs and Fg between the third and fourth gears 223, 227a and 227b and the strap 270, the strap 270 may not be released. However, a force Fout pulling out the strap 270 is greater than the frictional forces Fs and Fg between the third and fourth gears 223, 227a and 227b and the strap 270, the first gear 222 may be reversely rotated. That is, assuming that the frictional force between the third and fourth gears 223, 227a and 227b and the strap 270 is Fs and the force pulling the strap 270 out is Fout, the third gear 223 may be rotated in a state of Fout>Fs. Since the third gear 223 is formed on the first shaft 221 together with the first gear 222, the rotational force generated between the strap 270 and the third gear 223 is transferred to the first shaft 221. Accordingly, the first shaft 221 is rotated. The rotational force of the first shaft 221 is then transferred to the second shaft 224 by the first and second gears 222 and 225, and thereby the first and second gears 222 and 225 are also rotated in response to the rotation of the third gear 223. Through such processes, the strap 270 is moved in a backward direction which is opposite to a forward direction which is a direction that the strap 270 is inserted into the buckle unit 260.

The one embodiment disclosed herein proposes a structure of preventing the backward movement of the strap 270. That is, the buckle unit 260 according to the one embodiment disclosed herein has a structure for preventing the belt from being unexpectedly loosened.

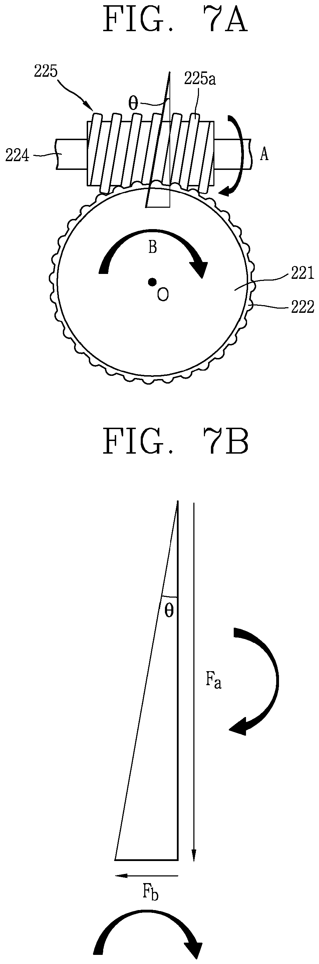

FIG. 7A is a view illustrating a shape that the first shaft 221 and the second shaft 224 intersect with each other in accordance with one embodiment disclosed herein, and FIG. 7B is a view schematically illustrating forces applied to the first shaft 221 and the second shaft 224.

As illustrated in FIGS. 7A and 7B, the first shaft 221 is rotated in a direction B and the second shaft 224 is rotated in a direction A. Accordingly, it can be noticed that a rotational shaft to which a force is transferred is changed from the second shaft 224 into the first shaft 221 by the engagement between the first gear 222 and the second gear 225. In this instance, if it is assumed that a force of rotating the second shaft 224 is Fa and a force of rotating the first shaft 221 is Fb, since Fa>Fb, the rotational force of the second shaft 224 is transferred to the first shaft 221. That is, the rotation of the second gear 225 driven by the driving motor 215 is transferred to the first gear 222, thereby controlling an amount of rotation of the first gear 222. Accordingly, when the strap 270 is loosely coupled, the strap 270 can be more tightened.

On the other hand, when manually manipulating the strap 270 in order to move the strap 270 in an opposite direction, the rotational force of the first gear 222 should be transferred to the second gear 225. In this instance, since Fb is weaker than Fa, the second gear 225 is not rotated by the first gear 222. That is, the second gear 225 is configured not to be backwardly rotated. Here, the backward rotation refers to that the second gear 225 is not rotated due to the rotation of the first gear 222. This means that the rotation of the second gear 225 is opposite to a forward rotation transferred by the first gear 222.

Meanwhile, the second gear 225 has a thread 225a formed in a spiral shape. The thread 225a should be tilted by less than 45° from a direction which is perpendicular to the second shaft 224. If exceeding 45°, the first gear 222 is not easily rotated even though the rotational force of the second gear 225 is transferred to the first gear 222. Preferably, a tilt angle θ of the thread 225a of the second gear 225 may be in the range of 15 to 20° in one embodiment disclosed herein. The angle θ may be referred to as a gear angle for convenience of explanation.

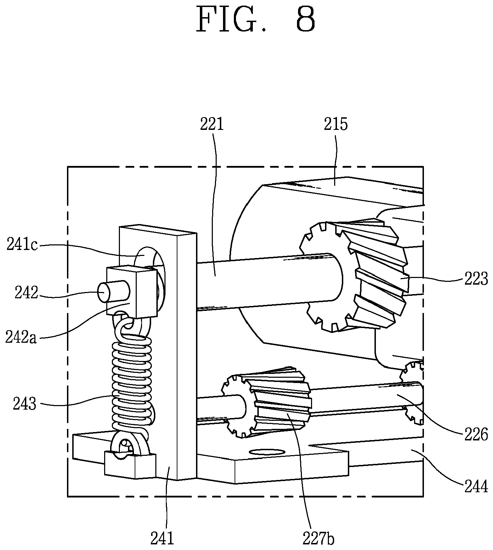

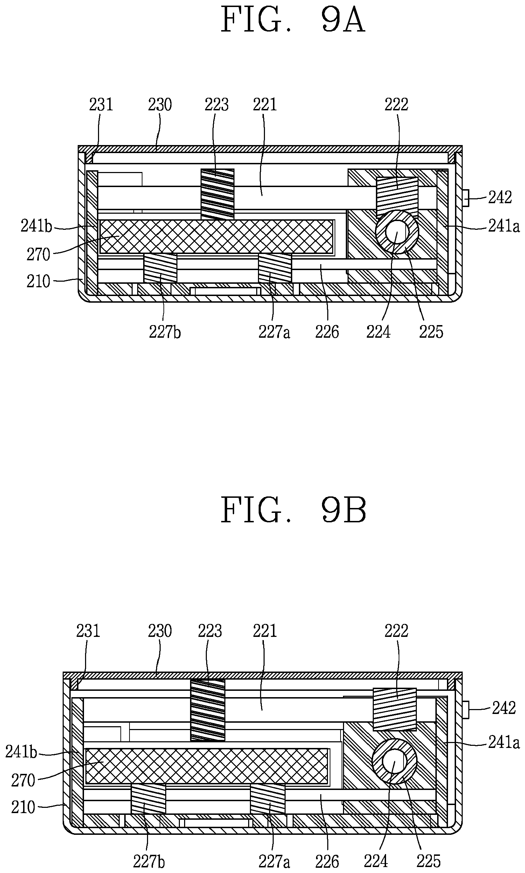

In this manner, the second gear 225 is difficult to be backwardly rotated, but required to be manually handled under an emergency. To this end, the smart belt 200 according to the one embodiment disclosed herein is provided with a slide button 242. FIG. 8 is a view illustrating a configuration of allowing a manual manipulation of the smart belt 200 according to one embodiment disclosed herein. FIG. 9A illustrates that the strap 270 is not movable due to being fixed by the gear unit 220, and FIG. 9B illustrates that the first shaft 221 has been moved upward by the slide button 242.

As illustrated in FIGS. 8, 9A and 9B, the smart belt 200 according to the one exemplary embodiment disclosed herein includes a supporting member 241, a slide button 242 and a first elastic member 243. The supporting member 241 is provided as a pair 241a and 241b. The pair of supporting members 241a and 241b are provided at both ends of the first shaft 221 and the third shaft 226 arranged in parallel to each other.

As illustrated in FIG. 8, one of the pair of supporting members 241a and 241b which is provided at one end side of the first shaft 221 and the third shaft 226 has a through hole 241c, through which the first shaft 221 is inserted to be externally exposed, and fixes one end portion of the third shaft 226. The slide button 242 is provided on at least one end portion of the first shaft 221 to selectively space the first gear 222 and the second gear 225 apart from each other. The first elastic member 243 is coupled to an end portion of the first shaft 221 so as to apply a restoring force to the first shaft 221.

That is, in an emergency condition or a discharge state of a battery 252, the smart belt 200 can be manually manipulated.

For the manual manipulation, when the slide button 242 is pulled up, the first shaft 221 connected to the slide button 242 is moved up along with the slide button 242. Accordingly, a gap between the third gear 223 and the fourth gears 227a and 227b increases more, which allows the strap 270 to be non-contactable with the third gear 223, thereby releasing a fastened state of the strap 270. In this state, the user can manually insert or draw out the strap 270.

The release of the strap 270 by the slide button 242 is temporary. Thus, as soon as the slide button 242 is set free, a restoring force is generated by the first elastic member 243 to restore the first shaft 221 to its original position.

In this instance, the first elastic member 243 may be implemented as a tension spring. The tension spring should have an elastic modulus which is not a burden when the user pulls the slide button 242. This means that the first elastic member 243 should apply an elastic force which is as strong as the slide button 242 being slidable only when the user pulls the slide button 242, without a malfunction, while the driving motor 215 operates. In the one embodiment disclosed herein, the elastic force of the first elastic member 243 in an idle state may be in the range of 1.5 to 2.0 kgf. The elastic force in the idle state refers to an elastic force in the first state.

If the first elastic member 243 is operated only when a force greater than 2.0 kgf is applied, the user may feel pressure. If the first shaft 221 is moved even when a force weaker than 1.5 kgf is applied, the first shaft 221 may be moved even when it is desired to secure the strap 270 by operating the driving motor 215. This may result in failing to fix the strap 270 .

Therefore, the first elastic member 243 in the one embodiment disclosed herein may be configured to operate when a force in the range of 1.5 to 2.0 kgf is applied. However, this is merely illustrative. The least force for operating the first elastic member 243 may be greater or smaller than 1.5 kgf and the greatest force therefor may also be greater than 2.0 kgf. In this instance, the slide button 242 provided on the end portion of the first shaft 221 is externally exposed through the through hole 241c. FIGS. 9A and 9B illustrate that the slide button 242 is provided only at one side, but the present invention may not be limited to this. The slide button 242 may, of course, be provided on both end portions of the first shaft 221.

In addition, a connection member 242a for connecting the slide button 242 and the first elastic member 243 is formed on the first shaft 221. The slide button 242 may also be operated in a pressing manner, but does not necessarily have to be pressed. Alternatively, the slide button 242 may protrude enough to be externally exposed such that the user can move it in one direction. Therefore, the slide button 242 may be made of a different material from the first shaft 221 or separately formed from the first shaft 221. However, it may be an end portion of the first shaft 221 which extends from the first shaft 221.

In this instance, as illustrated in FIGS. 9A and 9B, the third gear 223 is spaced apart from the first gear 222, and the first gear 222 and the second gear 225 are located in a space, which is spatially separated from an inserted space of the strap 270.

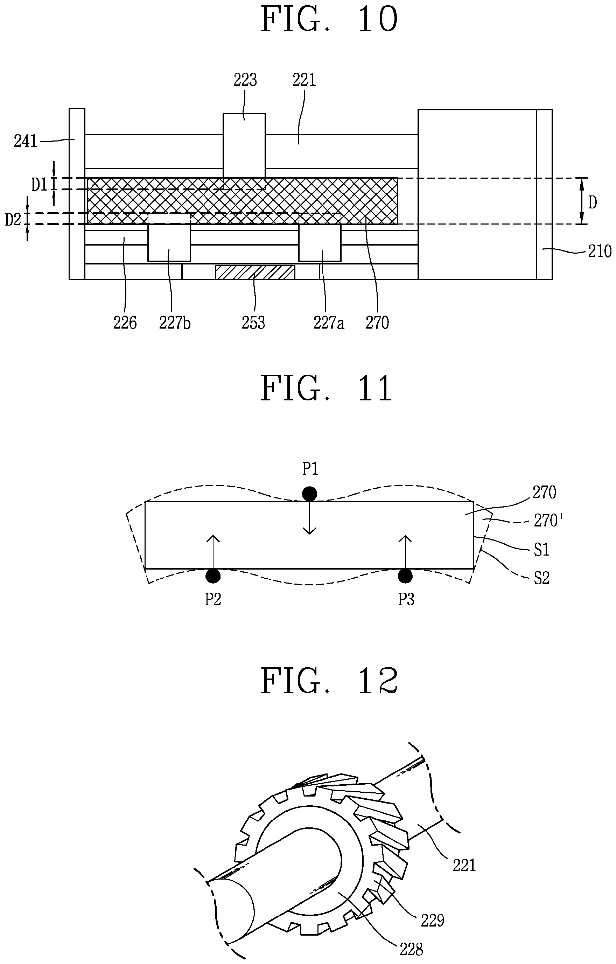

In addition, the third gear 223 and the fourth gears 227a and 227b which are gears contactable with the strap 270 may have outer portions which are preferably made of a material generating a great frictional force against the strap 270 in order to increase the frictional force against the strap 270. For example, as illustrated in FIG. 12, metal members 228 are formed on inner sides of the third gear 223 and the fourth gears 227a and 227b, respectively, and rubber members 229 are formed on outer circumferences of the metal members 228, respectively. For example, the metal member 228 which is easily formed may first be formed on the outer circumference of each of the first shaft 221 and the third shaft 226, and then the rubber member 229 may be formed on the outer surface of the metal member 228.

As such, the metal material may be disposed at the inner side and the rubber member 229 such as a tire of a vehicle may be disposed at the outer side. In this instance, the rubber member 229 may be formed in an intagliated pattern, like a shape of a vehicle wheel, and the intagliated pattern may be skewed with respect to the first shaft 221 and the third shaft 226. The metal member 228 may be made of stainless steel or aluminum. In this manner, the rubber member 229 may be tilted to increase the frictional force against the strap 270, which may result in preventing the strap 270 from being slid. Also, if the strap 270 is made of urethane, the frictional force between the strap 270 and the rubber member 229 can increase.

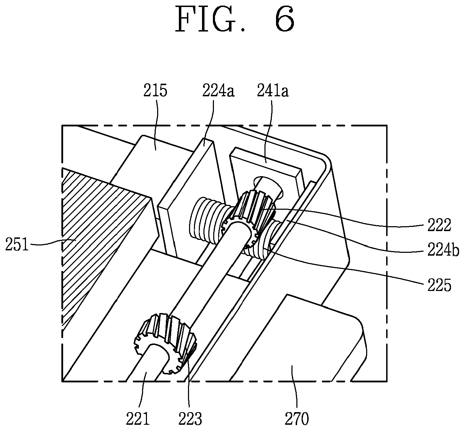

FIG. 5 is a view illustrating a state that the strap 270 is inserted into the buckle unit 260 in accordance with the one exemplary embodiment, and FIG. 6 is an enlarged perspective view of a region B of FIG. 5. Hereinafter, description will be given with reference to FIGS. 5 and 6, along with FIGS. 3 and 4A.

The smart belt 200 according to the one embodiment disclosed herein may further include a main circuit board 251 disposed within the body 210, a battery 252 disposed at one side of the main circuit board 251, and a movement-detecting sensor 253 (see FIG. 3) to detect the movement of the strap 270. The battery 252 is disposed in a battery mounting portion 252a. The gear unit 220 is disposed in gear mounting portions 220a and 220b which are stepped with each other (see FIG. 3). The gear unit 220 includes the driving motor 215 and the first to third shafts 221, 224 and 226. Since a formation thickness of the first to third shafts 221, 224 and 226 is thicker than that of the driving motor 215, the driving motor mounting portion 220b for mounting the driving motor 215 and the shaft mounting portion 220a are formed in the stepped manner. In this instance, the gear unit 220 is formed approximately in a shape like 'L' and thus the gear mounting portions 220a and 220b are also formed in a shape like 'L.'

Also, a vibration motor 261 as a haptic module may be provided in accordance with the one embodiment disclosed herein. Since the vibration motor 261 is disposed at one side of the battery 252, as illustrated in FIG. 4A, a vibration motor mounting portion 261a is formed adjacent to the battery mounting portion 252a. Also, an interface unit 262 through which data is transmitted or received or charging is enabled may be disposed on one of the side surfaces of the body 210. For this, a through hole 262a is formed.

The interface unit 262 serves as a path allowing the smart belt 200 to interface with various types of external devices connected thereto. The interface unit 262 may include at least one of wired or wireless ports, external power supply ports, wired or wireless data ports, memory card ports, ports for connecting a device having an identification module, audio input/output (I/O) ports, video I/O ports, earphone ports, and the like. In some cases, the smart belt 200 may perform assorted control functions associated with a connected external device, in response to the external device being connected to the interface unit 262.

The movement-detecting sensor 253 is a sensor which is provided adjacent to the through hole 211 formed through the side surface of the body 210 to detect the movement of the strap 270. The movement-detecting sensor 253 may be one of an image sensor 254, a hall sensor 255 or a resistance-measurement sensor 256.

FIGS. 15A to 15C illustrate a process that the strap 270 is inserted into the buckle unit 260, namely, illustrate a sensor for detecting an insertion of the strap 270. As illustrated in FIG. 15A, when the movement-detecting sensor 253 is the image sensor 254, whether or not the strap 270 moves close may be detected by a light-emitting portion and a light-receiving portion of the image sensor 254 provided in the buckle unit 260, and a moving distance can be calculated based on a moving speed of the strap 270. The image sensor 254 may use an infrared (IR) laser. For example, a laser beam is emitted from the light-emitting portion of the image sensor 254 toward the strap 270, and reflected on a surface of the strap 270. The reflected laser beam is incident onto the light-receiving portion. An inserted moment of the strap 270 can be detected by sensing the reflected light or laser beam, and the moving distance of the strap 270 can be calculated by detecting the moving speed and time of the strap 270.

Also, as illustrated in FIG. 15B, when the movement-detecting sensor 253 is the hall sensor 255, a plurality of magnetic members 255a and 255b are provided in the belt. The magnetic members 255a and 255b are disposed with a predetermined gap. The movement of the strap 270 is detected by sensing changes in magnetic fields by the magnetic members 255a and 255b.

In this instance, the plurality of magnetic members 255a and 255b may have the same polarity, or the magnetic members 255a and 255b having different polarities may be provided in an alternating manner. When the plurality of magnetic members 255a and 255b have the same polarity, the hall sensor 255 senses the change in the magnetic field by one magnetic member 255a, and thereafter senses a gradual reduction of strength of the magnetic field until the hall sensor 255 is located at a middle point between the one magnetic member 255a and another adjacent magnetic member 255b. Then, the hall sensor 255 senses the change in the magnetic field when the strength of the magnetic field is increased again by the adjacent magnetic member 255b. In this manner, the hall sensor 255 detects a number of the changes in the magnetic field. This may allow a detection of the moving distance of the strap 270. In this instance, the magnetic members 255a and 255b are disposed with a preset interval.

When the magnetic members 255a and 255b having the different polarities are disposed in the alternating manner, a number of changes in the polarities of the magnetic members 255a and 255b sensed by the hall sensor 255 may be detected, thereby calculating the moving distance of the strap 270.

In addition, as illustrated in FIG. 15C, when the movement-detecting sensor 253 is the resistance-measurement sensor 256, a conductive material 256a should be provided on the strap 270. That is, the conductive material 256a having resistance is patterned on the strap 270. Accordingly, the resistance-measurement sensor 256 may detect the resistance of the conductive material 256 when it is brought into contact with the conductive material 256a of the strap 270, thereby calculating the moving distance of the strap 270. For example, the conductive material 256a may be a variable resistor, and configured in a manner that the resistance is gradually increasing or decreasing from one end portion to another end portion. Also, the changes in the resistance may have a predetermined gradient, and a length of the conductive material 256a or the gradient of the resistance may be set to a predetermined value. The gradient of the resistance refers to the change in the resistance according to a position of the conductive material 256a, namely, a changed value of the resistance within a predetermined section.

As such, the movement and moving distance of the strap 270 can be detected in various manners by using such optical properties, magnetic field and conductivity. However, it is preferable that the movement-detecting method is implemented merely by the image sensor provided in the buckle unit 260, without a need of a separate structure equipped in the strap 270.

In this instance, an overlapped length of the strap 270 is calculated, and the overlapped length of the strap 270 is subtracted from a preset length of the strap 270, thereby calculating a user's waistline. The overlapped length of the strap 270 may also be calculated by multiplying a rotation speed (rpm), a rotation time and a diameter of the third gear 223.

Meanwhile, FIG. 14A illustrates a state before the strap 270 is inserted into the buckle unit 260 in accordance with the one exemplary embodiment, and FIG. 14B illustrates a state that the strap 270 has been inserted into the buckle unit 260 in accordance with the one exemplary embodiment.

As illustrated in FIGS. 14A and 14B, a door 213 which is rotatable toward an inner side of the body 210 is implemented as a pair of doors 213a and 213b provided at both end portions of the through hole 211, respectively. A pair of second elastic members 214a and 214b that apply restoring forces to the door 213 are provided at the pair of doors 213a and 213b, respectively. The pair of doors 213a and 213b may be referred to as first and second doors 213a and 213b. The first and second doors 213a and 213b are rotated up and down, respectively, thereby defining a space in which the strap 270 can be introduced into the body 210.

Also, after the strap 270 is inserted into the body 210, restoring forces should be applied to the first and second doors 213a and 213b in order to prevent an introduction of external light into the body 210 through the through holes 211 and 212. However, since the first and second doors 213a and 213b cannot be rotated to their positions in the first state due to the strap 270, the first and second doors 213a and 213b are configured to be adhered as closely as possible onto upper and lower surfaces of the strap 270, respectively. To this end, the pair of second elastic members 214a and 214b is needed. The pair of second elastic members 214a and 214b may be torsion springs, for example.

As such, by applying the restoring forces to the first and second doors 213a and 213b, an adverse effect to the operation of the image sensor 254 can be prevented.

FIG. 10 illustrates an arrangement of gears overlaid with the strap 270 in accordance with the one exemplary embodiment, and FIG. 11 is a schematic view illustrating positions of forces applied to the strap 270 in accordance with the one exemplary embodiment. As illustrated in FIGS. 10 and 11, the third gear 223 is closely adhered onto one surface of the strap 270, and the fourth gears 227a and 227b are closely adhered onto another surface of the strap 270. In this instance, if it is assumed that a thickness of the strap 270 is D, an overlapped thickness between the third gear 223 and the strap 270 is D1 and an overlapped thickness between the fourth gears 227a and 227b and the strap 270 is D2. Therefore, a gap between the third gear 223 and the fourth gears 227a and 227b is D-D1-D2. In this manner, in the one embodiment disclosed herein, at least part of the strap 270 is overlapped by the third gear 223 and the fourth gears 227a and 227b.

This is to stably support the strap 270, considering that the thickness of the strap 270 becomes uneven due to a processing deviation and a used state.

In this instance, the strap 270 is supported at three points P1, P2 and P3 which correspond to located points of the third gear 223 and the fourth gears 227a and 227b. The strap 270 may partially be curved at contact points with the third gear 223 and the fourth gears 227a and 227b. FIG. 11 illustrates the strap 270 in a state (S1) without being pressed by the third gear 223 and the fourth gears 227a and 227b, and a strap 270' in a state (S2) of being pressed by the third gear 223 and the fourth gears 227a and 227b. The strap 270 may be curved into a shape similar to 'S' when being pressed by the third gear 223 and the fourth gears 227a and 227b.

As described, in the one embodiment disclosed herein, the strap 270 is brought into contact with the third gear 223 and the fourth gears 227a and 227b at the three points P1, P2 and P3.

FIGS. 13A to 13C are sectional views illustrating the inserted state of the strap 270 according to an exemplary variation. As illustrated in FIG. 13A, if the third gear 223 is formed as a single roller, it comes in surface-contact with the strap 270 and a smooth operation of the third gear 223 is interrupted due to a frictional force generated between a wall surface and the strap 270. In this instance, the third gear 223 in the variation of FIG. 13A may correspond to the first gear 221 in the one exemplary embodiment.

Also, as illustrated in FIG. 13B, if a protrusion 216 formed on an inner side of the body 210 is added to a single roller structure in order to avoid such surface-contact, the protrusion 216 and the third gear 223 may interfere with the insertion of the strap 270. That is, the protrusion 216 may serve as a stopper and thereby interfere with the insertion of the strap 270.

As illustrated in FIG. 13C, if the third gear 223 and the fourth gear 227 are provided one by one, it is difficult to hold the strap 270. When the strap 270 overlaps the third gear 223 and the fourth gear 227, an excessive force may be likely to be applied to the strap 270.

Therefore, in the one embodiment disclosed herein, the strap 270 can be stably supported at the three points P1, P2 and P3 in the curved shape like 'S' even when the strap 270 overlaps those gears 223, 227a and 227b,.

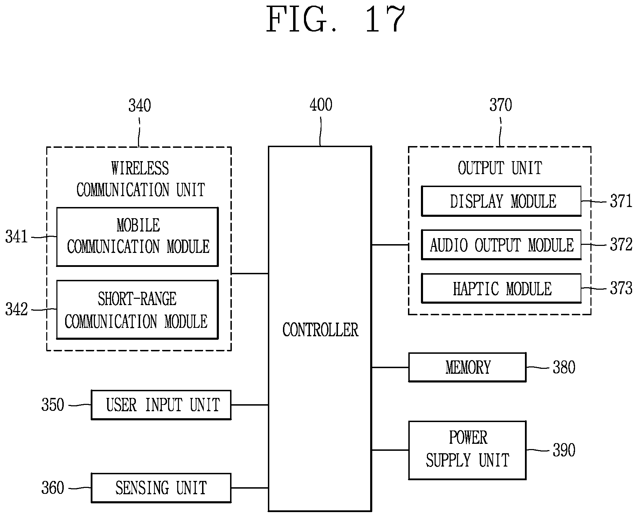

FIG. 17 is a view illustrating an internal configuration of the buckle unit 260 in accordance with one exemplary embodiment. As illustrated in FIG. 17, the buckle unit 260 according to the embodiment may include a part or all of the components of the mobile terminal illustrated in FIG. 1A. For example, as illustrated in FIG. 17, the buckle unit 260 according to the embodiment may include a wireless communication unit 340, a user input unit 350, a sensing unit 360, an output unit 370, a memory 380, a power supply unit 390 and a controller 400.

The wireless communication unit 340 may include a mobile communication module 341 and a short-range communication module 342. The short-range communication module 342 enables short-range communications including Wibro, WiFi, ZigBee, BluetoothTM, radio frequency (RF) and RFC.

The sensing unit 360 includes a plurality of sensors mounted in the smart belt 200 according to the one embodiment. The output unit 370 may include a display module 371 outputting various information, an audio output module 372 outputting an alert message or sound, and at least one haptic module 373 generating vibration for correcting a user's posture (guiding a proper posture). The driving motor 261 may be used as the haptic module 373.

The memory 380 may store posture information and movement (exercise) information related to a user sensed through the smart belt. The power supply unit 390 may be the battery 252, for example. The power supply unit 390 may supply power to the smart belt 200 and be charged with external power supplied through the interface unit 262.

The controller 400 controls a power-on/off of the sensors attached to the smart belt 200. The controller 400 may automatically adjust (change) an activity mode based on a change in a motion of the user's body and a change in a waist measurement (or waistline).

The controller 400 may decide an activity mode based on the user's motion, and control the smart belt 200 to tighten/loosen itself in response to the decided activity mode, thereby providing a different feedback to the user for each mode.

The controller 400 may control a power-on/off to the sensors attached to the smart belt 200 in a different manner according to the decided activity mode, thereby reducing power consumption due to a sensor operation. Also, the controller 400 may perform an assorted control by acquiring the user's position information through a built-in GPS sensor.

Hereinafter, operations of the smart belt 200 having such configuration according to the embodiment disclosed herein will be described with reference to the accompanying drawings.

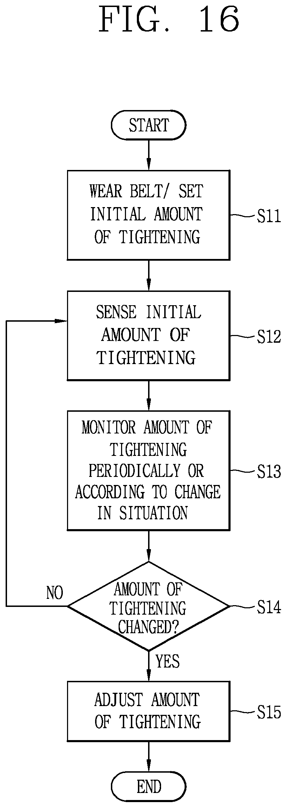

FIG. 16 is a flowchart illustrating a method of controlling the smart belt 200 in accordance with one embodiment of the present invention. Hereinafter, description will be given with reference to FIG. 16.

The smart belt 200 according to the one embodiment, as aforementioned, includes the buckle unit 260, the strap 270 movable through the buckle unit 260, and the controller 400 controlling an amount of tightening of the strap 270 by sensing a movement of the strap 270 and a force applied to the strap 270 through the plurality of sensors mounted in the buckle unit 260 and the strap 270.

An initial amount of tightening is set by the controller 400 (S11), and the initial amount of tightening is sensed (S12) so as to detect that the user has worn the smart belt 200. In this instance, the setting of the initial amount of tightening (S11) may alternatively be carried out before or after the smart belt 200 is worn.

Afterwards, an amount of tightening applied to the strap 270 is detected by the plurality of sensors (S13). When a change of the amount of tightening applied to the strap 270 is detected (S14), the amount of tightening is adjusted (S15). In this instance, the setting of the initial amount of tightening can be carried out in a manual manner because a preferred amount of tightening is different depending on users. In the one embodiment disclosed herein, the set amount of tightening is always maintained. For example, under assumption that the initial amount of tightening is set to 200 mN, when the amount of tightening is increased after the user has a meal, the strap 270 is loosened to maintain the initial amount of tightening. On the other hand, when a less amount than the initial amount of tightening is detected, the strap 270 is tightened to maintain the initial amount of tightening.

The plurality of sensors include a motion sensor detecting a user's motion, a gyro sensor detecting a user's moving direction, an acceleration sensor detecting a user's moving speed, a pressure sensor detecting internal pressure of the belt, and a tension sensor detecting a tensile force between the buckle unit 260 and the belt. The step of detecting the amount of tightening applied to the belt (S13) is carried out by a combination of at least one of the plurality of sensors. Also, the step of detecting the amount of tightening (S13) may be carried out in a periodic manner, by motion sensors S1, S2, S3 and S4 (see FIG. 2) or a GPS sensor.

The plurality of sensors, as illustrated in FIG. 2, may be provided within the buckle unit 260, but the present invention may not be limited to this. The plurality of sensors may alternatively be provided in one area of the strap 270. For example, the motion sensors and the gyro sensor of the plurality of sensors are arranged at plural points S1, S2, S3 and S4 of the smart belt 200 to detect the user's moving direction and a slouched posture, the tension sensor is arranged at a connection position S5 between the buckle unit 260 and the strap 270 to detect how strong the belt presses the user's waist, and the pressure sensors are evenly arranged within the strap 270 to detect pressure-applied positions of the strap 270. The sensors are basically disposed within the smart belt 200.

In this instance, the step of adjusting the amount of tightening (S15) may be carried out by the mobile terminal 100 connected to the smart belt 200.

The smart belt 200 according to the embodiment can be provided with a function of adjusting a tightened degree by automatically detecting a waist measurement of the user when the user wears the smart belt 200 on the waist.

In addition, the smart belt 200 according to the one embodiment can monitor the user's physical condition and posture based on the change in the length of the strap 270, and may be provided with a function of allowing the user to recognize the condition of the user's health by transmitting the physical condition and health-related information to the mobile terminal on the basis of the monitoring result.

To perform such functions, the smart belt 200 according to the one embodiment may be provided with the plurality of sensors detecting the user's physical condition or posture, and the driving motor 215 for adjusting the length of the strap 270. The sensors and the driving motor 215 may allow the strap 270 to automatically tighten or loosen itself according to the condition of the user's body in real time.

For example, when the user feels inconvenient due to a pants pressing a belly when sitting down, this physical state of the body (i.e., body state or body condition) can be detected through the sensors. In response to the detection of the body condition, the driving motor 215 provided in the buckle unit 260 operates to adjust the length of the strap 270. Accordingly, the smart belt 200 slightly loosens itself at the moment that the user wearing the smart belt 200 sits down and tightens itself back immediately when the person stands up. Also, if a belly temporarily protrudes after overeating, the strap 270 is automatically adjusted. Therefore, the user doesn't have to inconveniently adjust the length of the strap 270.

In addition, the smart belt 200 according to the one embodiment may be provided with a function of being operable with an application installed on the mobile terminal illustrated in FIGS. 1A to 1C. During the cooperation with the application, the user can set a tightened degree of the strap 270 that the user normally desires and limit the highest tightened and loosened degrees of the strap 270. According to this function, the smart belt 270 can check changes in the length of the strap 270 in real time and notify the checked result to the user such that the user can recognize excessive or non-excessive eating and the risk of obesity. When the user is in the risk of obesity, the smart belt 200 may transmit an alert message to the user not to increase the waist measurement any more. When it is determined that the user has gained weight, the smart belt 270 may provide health-related information (e.g., a type of exercise and a target of an exercise) by considering the user's height and weight.

Additionally, the smart belt 200 according to the one embodiment may be provided with at least one measurement device which is provided in the buckle unit 260, as well as the driving motor 215, to measure health-related information of the user. The measurement device may include an acceleration sensor for detecting information related to the user's motion.

The present invention proposes a method of providing a different feedback to the user for each mode by controlling the smart belt 200 to tighten/loosen itself according to a plurality of activity modes, in a manner of using the smart belt 200 having such various functions.

The plurality of activity modes may be set through a user menu. The user can set an amount of tightening/loosening of the strap 270 and positions of sensors to be powered on/off for each activity mode through a menu setting. If the user does not set the menu, the amount of tightening/loosening of the strap 270 and the positions of the sensors to be powered on/off are set as defaults for each activity mode. The types of activity modes and the setting according to the activity modes are merely illustrative, and the present invention may not be limited to them. The types of activity modes and the related setting may be combined, separated, or newly added according to the user's need.

In the one embodiment disclosed herein, the different feedback output through the smart belt 200 may include at least one of loosening of the length of the strap 270, or tightening of the length of the strap 270, and vibration/sound.

In addition, the present invention proposes a method of reducing power consumption due to sensor operations by differently controlling a power-on/off of the sensors, which are provided in the smart belt 200, for each activity mode, in a manner of using the smart belt 200 having such various functions.

The power-on/off of the sensors provided in the smart belt 200 is automatically controlled through determination of an activity mode on the basis of a change in a user's physical shape, such as the change in a motion of the user's body and the change in the user's waist measurement. The power-on/off of the sensors provided in the smart belt 200 may also be manually controlled according to a selection of a button provided on the smart belt 200 or a selection of an activity mode in the mobile terminal. In this instance, the smart belt 200 according to the one embodiment may be connected to the mobile terminal through a short-range communication network (e.g., Bluetooth, NFC, etc.).

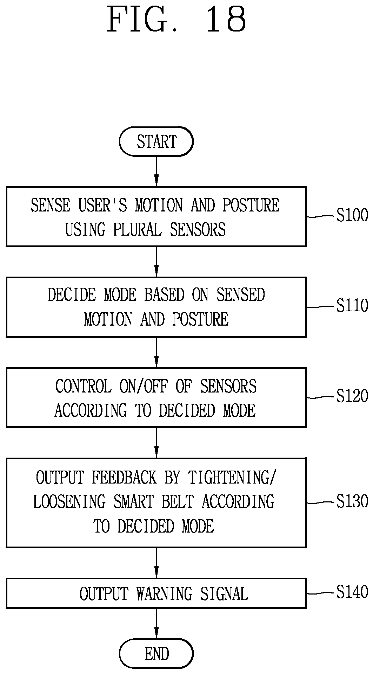

Hereinafter, a method of controlling the smart belt 200 according to one exemplary embodiment will be described in more detail, with reference to FIG. 18.

FIG. 18 is a flowchart illustrating an embodiment of using a smart belt in accordance with one embodiment of the present invention.

The controller 400 of the smart belt 200 according to the one embodiment may measure the user's motion and posture through the plurality of sensors when the user wears the smart belt 200 (S100). The user's motion and posture may include a moving speed/direction of the user's body, a tilt of the body, a change in a waist measurement, and a change in a tensile force of the belt. The plurality of sensors refer to every sensor mounted in the buckle unit 260 and the strap 270 of the smart belt 200, and may include a motion sensor, a gyro sensor, an acceleration sensor, a pressure sensor, a tensile sensor and a GPS sensor.

The controller 400 may decide a mode, namely, an activity mode indicating a user's current condition on the basis of the measured user's motion and posture (S110). The activity mode may roughly be divided into a working mode, a training (or exercise) mode, a diet mode and an eating mode. The working mode may be subdivided into a sitting/standing mode without a motion, and a walking mode with a motion, and the training mode may be subdivided into an aerobic exercise mode and an anaerobic exercise mode. The plurality of activity modes may be decided based on the user's motion, moving speed/direction, pressure/measurement of a waist, and the like, and be switched into one another.

Once the user's training mode is decided, the controller 400 may control the power-on/off of the sensors (S120), such that sensors which are not used in the decided training mode among the currently-operating plurality of sensors are powered off, thereby reducing power consumption. The power-on/off of the sensors according to the training mode is defined in advance and stored in the memory. The controller 400 may continuously measure the user's motion and posture through the currently-operating sensors.

Also, the controller 400 may control the driving motor 215 mounted in the buckle unit 260 of the smart belt 200 according to the decided mode to tighten/loosen the smart belt 200, and accordingly provide a different feedback to the user for each mode (S130). The feedback may be a guide feedback for inducing an effective motion according to the user's state. The feedback may be a feedback for correcting the user's wrong posture or guide information including the feedback for correcting the wrong posture and a solution thereof.

In addition, the controller 400 may output a warning signal when the user's posture is maintained almost the same as the previous state without being corrected after the output of the feedback (S140). The warning signal may be output in a form of at least one of sound, vibration and light, and transmitted to an external mobile terminal to be output thereby.

The controller 400 of the smart belt 200 according to the one embodiment may decide the user's activity mode in an automatic or manual manner. That is, the controller 400 may decide the training mode automatically on the basis of the user's moving speed/direction, a tilt of the body (waist), a change in a waist measurement, and a change in a tensile force of the belt, which are detected through the plurality of sensors. The memory may store reference data for deciding a mode. The controller 400 may determine the activity mode by comparing data measured through the sensors with the reference data stored in the memory. For example, when the user moves a lot or the length of the strap 270 changes by more than a predetermined length, the controller 400 may automatically decide the activity mode as the training mode.

Meanwhile, the controller 400 may control the smart belt 200 to tighten or loosen itself and the sensors to be powered on/off according to the decided training mode.

For example, an anaerobic (physical strength) exercise is an exercise of generating lactic acid and cannot be sustained for a long time. Therefore, the anaerobic exercise is an exercise which requires for high impact for a short time for which breathing is stopped. Therefore, breathing is very important in the anaerobic exercise such as muscle training. When muscles are contracted (positive) while holding a heavy machine, the user's body requires for an increased blood flow and overall blood pressure raises. Accordingly, the user should take a breath out to adjust the blood flow. On the other hand, if the user takes a breath in for the positive case and a breath out for a muscle relaxation (negative case), a power output or a pumping degree is drastically lowered.

Therefore, when the anaerobic exercise is decided based on the user's motion and posture, the controller 400 automatically switches the activity mode into an anaerobic exercise mode, and turns on the motion sensors S1, S2, S3 and S4 detecting the user's training posture, the pressure sensor S6 detecting the user's breathing based on internal pressure and a position of the strap 270, and the tensile sensor S5 detecting a tightened state of the belt by detecting the tensile force between the buckle unit 260 and the strap 270, while turning off the other sensors. Simultaneously, the controller 400 adjusts the tightening/loosening of the smart belt 200 according to the change in the user's posture to induce the user's breathing to be synchronized with muscle contraction/relaxation.

For example, the controller 400 may loosen the smart belt 200 upon pushing a dumbbell up (muscle contraction) to induce the user to breathe in. On the other hand, the controller 400 may tighten the smart belt 200 upon pushing the dumbbell down (muscle relaxation) to induce the user to breathe out. Also, when the user has to keep the back straight during performing a squat, the controller 400 may tighten the smart belt 200 to make the user keep the back straight, thereby guiding a training posture.

Meanwhile, the aerobic exercise is a cardiovascular endurance exercise through aerobic respiration, and thus may include running and cycling as exercises which can be performed with low intensity for an extended time. It is efficient to perform the aerobic exercise with intensity which is so low not to be breathless.

As another embodiment, when the aerobic exercise is decided based on the user's motion and posture, the controller 400 automatically switches the activity mode into an aerobic exercise mode. The controller 400 then turns on the pressure sensor S6 detecting breathing based on internal pressure and position of the belt, the tensile sensor S5 detecting the tensile force between the buckle unit 260 and the strap 270 and the GPS sensor detecting the user's position, while turning off the other sensors. Simultaneously, the controller 400 adjusts tightening/loosening of the smart belt 200 such that the user can take exercise for a long time with low intensity for an efficient aerobic exercise. For example, the controller 400 tightens the smart belt 200 to induce a speed reduction when the user's hyperventilation is sensed, and loosens the smart belt 200 to induce a speed increase when the user breathes too slow.

As another embodiment, when a posture exercise is decided based on the user's motion and posture, the controller 400 automatically switches the activity mode into a posture exercise mode. The controller 400 then turns on the motion sensors S1, S2, S3 and S4 detecting the user's training posture, the gyro sensor detecting the user's tilt, and the tensile sensor S5 detecting the tensile force between the buckle unit 260 and the strap 270 to sense an appropriate tightening/loosening value, while turning off the other sensors. Simultaneously, the controller 400 adjusts the tightening/loosening of the smart belt 200 for an efficient posture exercise. For example, the controller 400 may adjust the tightening and loosening of the smart belt 200 according to the user's motion and a tile of the user's backbone, and guide the user to correct a wrong posture in a vibrating manner when the user's wrong posture is detected.

When walking is decided based on the user's motion and posture, the controller 400 automatically switches the activity mode into a walking mode. The controller 400 then turns on the motion sensors S1, S2, S3 and S4 detecting shaking of the user's body and the gyro sensor detecting a shaken direction of the body, while turning off the other sensors. Simultaneously, the controller 400 may flexibly adjust the tightening/loosening of the smart belt 200 according to the user's shaking.

When a wrong posture is detected according to the sensing results of the motion sensors and the gyro sensor with respect to a forwardly-bent degree of the user's body and left and right movements of the user's body, the controller 400 tightens the smart belt 200 and simultaneously outputs vibration to feed back on the wrong posture. The feedback is repetitively carried out for a predetermined time. If the user's wrong posture is not corrected after the predetermined time, the controller 400 may transmit a warning signal and guide information to the mobile terminal. The mobile terminal may output a warning sound and also output a posture guide on the display unit according to the warning signal.

When the sitting/standing mode is decided based on the user's motion and posture, the controller 400 automatically switches the activity mode into the sitting/standing mode. The controller 400 then turns on the motion sensors S1, S2, S3 and S4 detecting a bent degree of the user's back and the tension sensor S5 detecting the tensile force between the buckle unit 250 and the strap 270 to sense an appropriate tightening/loosening value, while turning off the other sensors. Simultaneously, the controller 400 detects a forwardly-bent degree of the user's body and a twisted degree of the body to left or right using the motion sensors S1, S2, S3 and S4, compares the sensed values with a prestored posture value, and checks whether or not the user currently takes a correct sitting/standing posture.

Therefore, the controller 400 reads out an appropriate tightening/loosening value from the memory on the basis of the tensile force sensed through the tension sensor S5 according to a sitting posture and a standing posture, thereby flexibly adjusting the tightening/loosening of the smart belt 200. When the user's posture is wrong, the controller 400 may output a posture correcting feedback with respect to the sitting/standing posture. For example, the controller 400 loosens the smart belt 200 when the waist becomes thick due to the user's sitting posture, and tightens the smart belt 200 when the waist becomes thin due to the user's standing posture. Also, the controller 400 guides the user to properly straighten the back by tightening the smart belt 200 when the user sits/stands slouched.

In addition, when the user's bent back and curved backbone are sensed based on the sensing results of the motion sensors S1, S2, S3 and S4, the controller 400 may output a feedback on a correct posture. The feedback may be carried out in a manner of tightening the smart belt 200 or in a vibrating manner. For example, the controller 400 may guide the user to take good posture by continuously tightening the smart belt 200 until the user takes such good posture. When the user sits with the backbone slouched, the controller 400 outputs vibration through the vibration motor 261, which is located in a direction that the backbone is slouched, so as for the user to take good posture. The controller 400 compares a bent or curved angle of the user's body with a reference value to correct the user's posture. The controller 400 loosens the belt or weakly outputs vibration as getting close to the reference value.

The present invention proposes a method of sharing a user's sitting/standing behavior with other users in accordance with one exemplary embodiment. When this sharing method is used, the user's living habits can be easily compared with other users and problems of the user's lifestyle can be recognized. Accordingly, when the user goes to the doctor, the user can give correct data associated with the user's habits to the doctor without relying on memorized data.

In general, a waist measurement changes after having a meal or according to motions. For example, the waist measurement increases from 1.1 cm to 2.9 cm on the average when the user stands up straight, sits on a chair or kneels down, and increases by about 1.5 cm after having a meal.

Therefore, the present invention provides a method of feeding back on an amount of food intake and guiding a posture after a meal by detecting the user's waist measurement using the smart belt 200.

For example, when an eating mode is decided based on the user's position, motion and posture, the controller 400 automatically switches the activity mode into the eating mode. The controller 400 then turns on the motion sensors S1, S2, S3 and S4 detecting the user's posture, the tension sensor S5 detecting the tensile force between the buckle unit 260 and the strap 270 to sense an appropriate tightening/loosening value, and the image sensor 254 detecting the waist measurement, while turning off the other sensors. The controller 400 can sense an increased amount of the waist measurement after a meal, a loosened degree of the smart belt 200 and a posture after a meal.

The controller 400 checks whether or not the user's belly expands or not by detecting the waist measurement during a meal on the basis of an output of the image sensor 254 or a loosened amount of the belt. If the waist measurement exceeds a predetermined threshold value, the controller 400 may feed back on the risk of overeating through the output unit or by transmitting a warning signal to the mobile terminal.

In this instance, when the smart belt 200 is connected to the mobile terminal 100 through Bluetooth, it can be sensed that the user is located in a restaurant, and accordingly the smart belt 200 can automatically check and adjust the amount of tightening.

When the belly expansion due to overeating is sensed, the controller 400 tightens the smart belt 200 to provide feedback such that the user avoids overeating. Afterwards, a tightened state of the belt is maintained at a degree that is not too tight or too loose.

In general, the living habit of sitting for a long time increases a risk of death by about 15-20%, and risks of cardiac disorder, cancers and diabetes by 90% to the maximum. Therefore, when the sitting posture is sensed for a long time, it is important to feed back on such risks.

In addition, the user's waist can continuously be tracked (checked, monitored), which may enable the user to be in a good shape. For example, when the user's waist measurement is suddenly increased, a message, such as "Your waistline has drastically increased recently. You need to reduce the waistline. Squat and sit-ups are recommended," may be output.

As described above, according to the present invention, the activity mode may be set by detecting the user's posture and motion through the plurality of sensors provided in the smart belt 200, tightening/loosening of the smart belt 200 can be controlled according to the set activity mode so as to provide a different feedback to the user for each mode. This may result in providing an effect of notifying accurate information suitable for a current condition or correcting the user's posture.

The present invention can be implemented as computer-readable codes in a program-recorded medium. The computer-readable medium may include all types of recording devices each storing data readable by a computer system. Examples of such computer-readable media may include hard disk drive (HDD), solid state disk (SSD), silicon disk drive (SDD), ROM, RAM, CD-ROM, magnetic tape, floppy disk, optical data storage element and the like. Also, the computer-readable medium may also be implemented as a format of carrier wave (e.g., transmission via an Internet). The computer may include the controller 180 of the terminal. Therefore, it should also be understood that the above-described embodiments are not limited by any of the details of the foregoing description, unless otherwise specified, but rather should be construed broadly within its scope as defined in the appended claims, and therefore all changes and modifications that fall within the metes and bounds of the claims, or equivalents of such metes and bounds are therefore intended to be embraced by the appended claims.

## Claims

1. A smart belt (200) comprising:
a buckle unit (260);
a strap (270) movable through the buckle unit(260);
a motion sensor (S1, S2, S3, S4) configured to detect a user's motion or posture;
a controller (400) configured to control an amount of tightening of the strap (270) by detecting a movement of the strap(270) and a force applied to the strap (270);
a body (210) having a front surface, a rear surface opposite the front surface, and side surfaces extending between the front surface and the rear surface, the body (210) being configured such that the strap (270) is insertable through a through hole (211, 212) in one of the side surfaces in a first direction;
a cover (230) configured to cover the rear surface of the body (210);
a movement-detecting sensor (253) configured to detect a movement of the strap (270) and being disposed adjacent to the through hole (211); and
a gear unit (220) provided in the body (210), the gear unit (220) being configured to adjust an amount of tightening of the strap (270) by displacing the strap (270) along the first direction, the gear unit (220) including:
a first shaft (221) extending in a second direction intersecting the first direction, the first shaft (221) being provided with a first gear (222), and the first shaft (221) is provided with a third gear (223) spaced apart from the first gear (222) by a first predetermined gap;
a second shaft (224) extending in a second direction, the second direction intersecting the first shaft (221), and the second shaft (224) being provided with a second gear (222, 225) to engage the first gear (222); and
a third shaft (226) extending parallel to the first shaft (221) spaced apart from the first shaft (221) by a second predetermined gap, and the third shaft (226) being provided with a pair of fourth gears (227a,227b),
wherein a rotation force of the second shaft (224) is configured to be transferred to the first shaft (221) through engagement of the first gear (222) and the second gear (222, 225),
wherein the strap (270) is insertable between the third gear (223) and the pair of fourth gears (227a, 227b) and the amount of tightening of the strap (270) is adjustable via contact with the third gear (223) and the pair of fourth gears (227a, 227b),
wherein the third shaft (226) has opposite ends fixed to inner surfaces of the body (210),
wherein the strap (270) is arranged to overlap the third gear (223) and the pair of fourth gears (227a, 227b) when inserted between the third gear (223) and the pair of fourth gears (227a, 227b), and
wherein the third gear (223) is arranged between the pair of fourth gears (227a, 227b), and
wherein each gear of the third gear (223) and the pair of fourth gears (227a, 227b) comprises:
a metal member (228) provided on outer circumferences of the first shaft (221) and the third shaft (226), respectively; and
a rubber member (229) provided on an outer circumference of the metal member (228).

2. The smart belt of claim 1, wherein the second shaft (224) is connected to a motor (215) to rotate the second shaft (224),
wherein the second gear (222, 225) is a worm gear, and
wherein the first gear (222) is a spur gear configured to engage the second gear (222, 225).

3. The smart belt of claim 2, wherein the second gear (222, 225) is provided with a spiral thread, and an angle of the spiral thread is less than 45° from a direction perpendicular to the second shaft (224).

4. The smart belt of claim 1, further comprising:
a pair of supporting members (241a, 241b) provided at opposite end portions of the first shaft (221) and opposite end portions of the third shaft (226), one supporting member of the pair of supporting members (241a, 241b) has a through hole (241c) formed therethrough such that the first shaft (221) is inserted through the through hole (241c) to be externally exposed, and the one supporting member fixes one end portion of the opposite end portions of the of the third shaft (226);
a slide button (242) provided on an end portion of the opposite end portions of the first shaft (221), the slide button (242) configured to selectively separate the first gear (222) and the second gear (222, 225) from each other; and
a first elastic member (243) provided on the end portion of the opposite end portions of the first shaft (221), the first elastic member (243) being configured to apply a restoring force to the first shaft (221).

5. The smart belt of claim 1, wherein the cover (230) includes a protrusion (231) protruding from one surface thereof toward the body (210), and
wherein the protrusion (231) contacts an inner surface of the body (210).

6. The smart belt of claim 1, further comprising:
a main circuit board (251) in the body (210);
a battery (252) located at one side of the main circuit board (251); and
a vibration motor (261) located at one side of the battery (252), the vibration motor (261) configured to generate vibration.

7. The smart belt of claim 6, wherein the movement-detecting sensor (253) is one of an image sensor, a hall sensor (255) or a resistance-measurement sensor,
wherein the strap (270) is provided with magnetic members (255a, 255b) when the movement-detecting sensor (253) is the hall sensor (255), and
wherein the strap (270) is provided with a conductive member when the movement-detecting sensor (253) is the resistance-measurement sensor.

8. The smart belt of claim 6, further comprising:
a pair of doors (213a, 213b) coupled to opposite sides of the through hole (211), the pair of doors (213a, 213b) being rotatable away from each other toward an inner surface of the body (210); and
a second elastic member (214a, 214b) provided at each door of the pair of doors to apply a restoring force to said door.

9. The smart belt of claim 8, wherein the second elastic member (214a, 214b) is a torsion spring.

## Patentansprüche

1. Smart-Gürtel (200), der aufweist:
eine Schnalleneinheit (260);
einen Riemen (270), der durch die Schnalleneinheit (260) beweglich ist;
einen Bewegungssensor (S1 S2, S3, S4), der so konfiguriert ist, dass er die Bewegung oder Haltung eines Benutzers detektiert;
eine Steuerung (400), die so konfiguriert ist, dass sie einen Festziehbetrag des Riemens (270) steuert, indem sie eine Bewegung des Riemens (270) und eine auf den Riemen (270) ausgeübte Kraft detektiert;
einen Körper (210) mit einer Vorderfläche, einer Rückfläche entgegengesetzt zur Vorderfläche und Seitenflächen, die sich zwischen der Vorderfläche und der Rückfläche erstrecken, wobei der Körper (210) so konfiguriert ist, dass der Riemen (270) durch ein Durchgangsloch (211, 212) in einer der Seitenflächen in einer ersten Richtung einführbar ist;
einen Deckel (230), der so konfiguriert ist, dass er die Rückfläche des Körpers (210) abdeckt;
einen Bewegungsdetektionssensor (253), der so konfiguriert ist, dass er eine Bewegung des Riemens (270) detektiert, und benachbart zum Durchgangsloch (211) angeordnet ist; und
eine Zahnradeinheit (220), die im Körper (210) vorgesehen ist, wobei die Zahnradeinheit (220) so konfiguriert ist, dass sie einen Festziehbetrag des Riemens (270) einstellt, indem sie den Riemen (270) entlang der ersten Richtung verschiebt, wobei die Zahnradeinheit (220) aufweist:
eine erste Welle (221), die sich in einer zweiten Richtung erstreckt, die die erste Richtung schneidet, wobei die erste Welle (221) mit einem ersten Zahnrad (222) versehen ist und die erste Welle (221) mit einem dritten Zahnrad (223) versehen ist, das vom ersten Zahnrad (222) um eine erste vorbestimmte Lücke beabstandet ist;
eine zweite Welle (224), die sich in einer zweiten Richtung erstreckt, wobei die zweite Richtung die erste Welle (221) schneidet und die zweite Welle (224) mit einem zweiten Zahnrad (222, 225) versehen ist, um in das erste Zahnrad (222) einzugreifen; und
eine dritte Welle (226), die sich parallel zur ersten Welle (221) um eine zweite vorbestimmte Lücke von der ersten Welle (221) beabstandet erstreckt, und wobei die dritte Welle (226) mit einem Paar vierter Zahnräder (227a, 227b) versehen ist,
wobei eine Drehkraft der zweiten Welle (224) so konfiguriert ist, dass sie zur ersten Welle (221) über Eingriff des ersten Zahnrads (222) und des zweiten Zahnrads (222, 225) übertragen wird,
wobei der Riemen (270) zwischen dem dritten Zahnrad (223) und dem Paar vierter Zahnräder (227a, 227b) einführbar ist und der Festziehbetrag des Riemens (270) über Kontakt mit dem dritten Zahnrad (223) und dem Paar vierter Zahnräder (227a, 227b) einstellbar ist,
wobei die dritte Welle (226) entgegengesetzte Enden hat, die an Innenflächen des Körpers (210) befestigt sind,
wobei der Riemen (270) so angeordnet ist, dass er das dritte Zahnrad (223) und das Paar vierter Zahnräder (227a, 227b) überlappt, wenn er zwischen dem dritten Zahnrad (223) und dem Paar vierter Zahnräder (227a, 227b) eingeführt ist, und
wobei das dritte Zahnrad (223) zwischen dem Paar vierter Zahnräder (227a, 227b) angeordnet ist und
wobei jedes Zahnrad des dritten Zahnrads (223) und des Paars vierter Zahnräder (227a, 227b) aufweist:
ein Metallteil (228), das auf Außenumfängen der ersten Welle (221) bzw. der dritten Welle (226) vorgesehen ist; und
ein Gummiteil (229), das auf einem Außenumfang des Metallteils (228) vorgesehen ist.

2. Smart-Gürtel nach Anspruch 1, wobei die zweite Welle (224) mit einem Motor (215) verbunden ist, um die zweite Welle (224) zu drehen,
wobei das zweite Zahnrad (222, 225) ein Schneckenrad ist und
wobei das erste Zahnrad (222) ein Stirnrad ist, das so konfiguriert ist, dass es in das zweite Zahnrad (222, 225) eingreift.

3. Smart-Gürtel nach Anspruch 2, wobei das zweite Zahnrad (222, 225) mit einem Spiralgewinde versehen ist und ein Winkel des Spiralgewindes kleiner als 45° aus einer Richtung senkrecht zur zweiten Welle (224) ist.

4. Smart-Gürtel nach Anspruch 1, der ferner aufweist:
ein Paar Stützteile (241a, 241b), die an entgegengesetzten Endabschnitten der ersten Welle (221) und entgegengesetzten Endabschnitten der dritten Welle (226) vorgesehen sind, wobei ein Stützteil des Paars Stützteile (241a, 241b) ein Durchgangsloch (241c) hat, das so durchgehend gebildet ist, dass die erste Welle (221) durch das Durchgangsloch (241c) eingeführt ist, um außen freizuliegen, und das eine Stützteil einen Endabschnitt der entgegengesetzten Endabschnitte der dritten Welle (226) befestigt;
einen Schiebeknopf (242), der auf einem Endabschnitt der entgegengesetzten Endabschnitte der ersten Welle (221) vorgesehen ist, wobei der Schiebeknopf (242) so konfiguriert ist, dass er das erste Zahnrad (222) und das zweite Zahnrad (222, 225) selektiv voneinander trennt; und
ein erstes elastisches Teil (243), das auf dem Endabschnitt der entgegengesetzten Endabschnitte der ersten Welle (221) vorgesehen ist, wobei das erste elastische Teil (243) so konfiguriert ist, dass es eine Rückstellkraft auf die erste Welle (221) ausübt.

5. Smart-Gürtel nach Anspruch 1, wobei der Deckel (230) einen Vorsprung (231) aufweist, der von einer Oberfläche davon zum Körper (210) vorsteht, und
wobei der Vorsprung (231) eine Innenfläche des Körpers (210) kontaktiert.

6. Smart-Gürtel nach Anspruch 1, der ferner aufweist:
eine Hauptleiterplatte (251) im Körper (210);
eine Batterie (252), die an einer Seite der Hauptleiterplatte (251) liegt; und
einen Schwingungsmotor (261), der an einer Seite der Batterie (252) liegt, wobei
der Schwingungsmotor (261) so konfiguriert ist, dass er Schwingung erzeugt.

7. Smart-Gürtel nach Anspruch 6, wobei der Bewegungsdetektionssensor (253) ein Bildsensor, ein Hall-Sensor (255) oder ein Widerstandsmesssensor ist,
wobei der Riemen (270) mit Magnetteilen (255a, 255b) versehen ist, wenn der Bewegungsdetektionssensor (253) der Hall-Sensor (255) ist, und
wobei der Riemen (270) mit einem leitenden Teil versehen ist, wenn der Bewegungsdetektionssensor (253) der Widerstandsmesssensor ist.

8. Smart-Gürtel nach Anspruch 6, der ferner aufweist:
ein Paar Klappen (213a, 213b), die mit entgegengesetzten Seiten des Durchgangslochs (211) gekoppelt sind, wobei das Paar Klappen (213a, 213b) voneinander weg zu einer Innenfläche des Körpers (210) drehbar ist; und
ein zweites elastisches Teil (214a, 214b), das an jeder Klappe des Paars Klappen vorgesehen ist, um eine Rückstellkraft auf die Klappe auszuüben.

9. Smart-Gürtel nach Anspruch 8, wobei das zweite elastische Teil (214a, 214b) eine Torsionsfeder ist.

## Revendications

1. Ceinture intelligente (200) comprenant :
une unité de boucle (260) ;
une sangle (270) mobile à travers l'unité de boucle (260) ;
un capteur de mouvement (S1 S2, S3, S4) configuré pour détecter un mouvement ou une posture d'un utilisateur ;
un dispositif de commande (400) configuré pour commander une quantité de serrage de la sangle (270) en détectant un déplacement de la sangle (270) et une force appliquée à la sangle (270) ;
un corps (210) ayant une surface avant, une surface arrière à l'opposé de la surface avant, et des surfaces latérales s'étendant entre la surface avant et la surface arrière, le corps (210) étant configuré de sorte que la sangle (270) puisse être insérée à travers un trou traversant (211, 212) dans l'une des surfaces latérales dans une première direction ;
un couvercle (230) configuré pour couvrir la surface arrière du corps (210) ;
un capteur de détection de déplacement (253) configuré pour détecter un déplacement de la sangle (270) et disposé adjacent au trou traversant (211) ; et
une unité d'engrenage (220) prévue dans le corps (210), l'unité d'engrenage (220) étant configurée pour ajuster une quantité de serrage de la sangle (270) en déplaçant la sangle (270) suivant la première direction, l'unité d'engrenage (220) comportant :
un premier arbre (221) s'étendant dans une deuxième direction coupant la première direction, le premier arbre (221) étant pourvu d'un premier engrenage (222), et le premier arbre (221) étant pourvu d'un troisième engrenage (223) espacé du premier engrenage (222) d'un premier écartement prédéterminé ;
un deuxième arbre (224) s'étendant dans une deuxième direction, la deuxième direction coupant le premier arbre (221), et le deuxième arbre (224) étant pourvu d'un deuxième engrenage (222, 225) pour s'engrener avec le premier engrenage (222) ; et
un troisième arbre (226) s'étendant parallèlement au premier arbre (221) espacé du premier arbre (221) d'un deuxième écartement prédéterminé, et le troisième arbre (226) étant pourvu d'une paire de quatrièmes engrenages (227a, 227b),
dans laquelle une force de rotation du deuxième arbre (224) est configurée pour être transférée au premier arbre (221) par l'engrènement du premier engrenage (222) et du deuxième engrenage (222, 225),
dans laquelle la sangle (270) peut être insérée entre le troisième engrenage (223) et la paire de quatrièmes engrenages (227a, 227b) et la quantité de serrage de la sangle (270) peut être ajustée via un contact avec le troisième engrenage (223) et la paire de quatrièmes engrenages (227a, 227b),
dans laquelle le troisième arbre (226) a des extrémités opposées fixées à des surfaces intérieures du corps (210),
dans laquelle la sangle (270) est agencée pour chevaucher le troisième engrenage (223) et la paire de quatrièmes engrenages (227a, 227b) lorsqu'elle est insérée entre le troisième engrenage (223) et la paire de quatrièmes engrenages (227a, 227b), et
dans laquelle le troisième engrenage (223) est agencé entre la paire de quatrièmes engrenages (227a, 227b), et
dans laquelle chaque engrenage du troisième engrenage (223) et de la paire de quatrièmes engrenages (227a, 227b) comprend :
un organe en métal (228) prévu sur des circonférences extérieures du premier arbre (221) et du troisième arbre (226), respectivement ; et
un organe en caoutchouc (229) prévu sur une circonférence extérieure de l'organe en métal (228).

2. Ceinture intelligente selon la revendication 1, dans laquelle le deuxième arbre (224) est raccordé à un moteur (215) pour faire tourner le deuxième arbre (224),
dans laquelle le deuxième engrenage (222, 225) est une vis sans fin, et
dans laquelle le premier engrenage (222) est un engrenage droit configuré pour s'engrener avec le deuxième engrenage (222, 225).

3. Ceinture intelligente selon la revendication 2, dans laquelle le deuxième engrenage (222, 225) est pourvu d'un fil hélicoïdal, et un angle du fil hélicoïdal est inférieur à 45° depuis une direction perpendiculaire au deuxième arbre (224).

4. Ceinture intelligente selon la revendication 1, comprenant en outre :
une paire d'organes de support (241a, 241b) prévus au niveau de portions d'extrémité opposées du premier arbre (221) et de portions d'extrémité opposées du troisième arbre (226), un organe de support de la paire d'organes de support (241a, 241b) possède un trou traversant (241c) formé au travers de sorte que le premier arbre (221) soit inséré à travers le trou traversant (241c) pour être exposé à l'extérieur, et l'organe de support fixe une portion d'extrémité des portions d'extrémité opposées du troisième arbre (226) ;
un bouton coulissant (242) prévu sur une portion d'extrémité des portions d'extrémité opposées du premier arbre (221), le bouton coulissant (242) étant configuré pour séparer sélectivement le premier engrenage (222) et le deuxième engrenage (222, 225) l'un de l'autre ; et
un premier organe élastique (243) prévu sur la portion d'extrémité des portions d'extrémité opposées du premier arbre (221), le premier organe élastique (243) étant configuré pour appliquer une force de rappel au premier arbre (221).

5. Ceinture intelligente selon la revendication 1, dans laquelle le couvercle (230) comporte une protubérance (231) dépassant d'une de ses surfaces vers le corps (210), et
dans laquelle la protubérance (231) entre en contact avec une surface intérieure du corps (210).

6. Ceinture intelligente selon la revendication 1, comprenant en outre :
une carte de circuit principale (251) dans le corps (210) ;
une batterie (252) située d'un côté de la carte de circuit principale (251) ; et
un moteur de vibration (261) situé d'un côté de la batterie (252), le moteur de vibration (261) étant configuré pour générer une vibration.

7. Ceinture intelligente selon la revendication 6,
dans laquelle le capteur de détection de déplacement (253) est l'un parmi un capteur d'image, un capteur à effet hall (255) ou un capteur de mesure de résistance,
dans laquelle la sangle (270) est pourvue d'organes magnétiques (255a, 255b) lorsque le capteur de détection de déplacement (253) est le capteur à effet hall (255), et
dans laquelle la sangle (270) est pourvue d'un organe conducteur lorsque le capteur de détection de déplacement (253) est le capteur de mesure de résistance.

8. Ceinture intelligente selon la revendication 6, comprenant en outre :
une paire de trappes (213a, 213b) couplées à des côtés opposés du trou traversant (211), la paire de trappes (213a, 213b) pouvant tourner en éloignement l'une de l'autre vers une surface intérieure du corps (210) ; et
un deuxième organe élastique (214a, 214b) prévu au niveau de chaque trappe de la paire de trappes pour appliquer une force de rappel à ladite trappe.

9. Ceinture intelligente selon la revendication 8,
dans laquelle le deuxième organe élastique (214a, 214b) est un ressort de torsion.
